# EUROPÄISCHE PATENTANMELDUNG

(11) **EP 1 078 929 A2**
(43) Veröffentlichungstag der Anmeldung: **28.02.2001**
(21) Anmeldenummer: 00810732.8
(22) Anmeldetag: 17.08.2000
(51) Int. Cl.: C07D 498/10, C08K 5/353

(54) **Neue sterisch gehinderte Amine**

(30) Priorität: 18.08.1999 CH 151899
(71) Anmelder: Clariant Finance (BVI) Limited, Road Town, Tortola (VG)
(72) Erfinder: Stährfeldt, Thomas, 79576 Weil am Rhein (DE); Kröhnke, Christoph, 79206 Breisach-Oberrimsingen (DE); Wilbert, Götz, 86368 Gersthofen (DE)
(74) Vertreter: D'haemer, Jan Constant

(57) **Zusammenfassung**

Die vorliegende Erfindung bezieht sich auf sterisch gehinderte Amine als Stabilisatoren für organisches Material, insbesondere für Polymere, sowie auf Mischungen mit anderen Stabilisatoren.

Die neuen Stabilisatoren sowie Mischungen daraus mit anderen Stabilisatoren zeigen eine ausgezeichnete Schutzwirkung gegen den schädlichen Einfluss von Strahlung, Licht oder Wärme auf das zu schützende organische Material.

## Beschreibung

Die vorliegende Erfindung bezieht sich auf sterisch gehinderte Amine als Stabilisatoren für organisches Material, insbesondere für Polymere, sowie auf Mischungen mit anderen Stabilisatoren.

Es ist bekannt, dass organische Materialien durch Licht, Strahlung, Wärme oder Sauerstoff geschädigt werden. Es gibt bereits viele Druckschriften, die Verbindungen zur Stabilisierung von organischem Material gegen diese Einflüsse beschreiben. Es handelt sich dabei meist um Radikalfänger und insbesonders im Zusammenhang mit Lichtstabilisatoren um Verbindungen, die in der Lage sind, Hydroperoxide zu zersetzen, Quencher (Löscher für angeregte Zustände) oder um UV-Absorber. Bei Wärmestabilisatoren handelt es sich oft um sterisch gehinderte Phenole. Bei den Stabilisatoren, die einen Schutz gegen den thermischen Abbau im Verlauf der Verarbeitung insbesonders für organische Polymere bieten, handelt es sich oft um Phosphite oder Phosphonite (vgl. R. Gächter, H. Müller, Plastics Additives Handbook, 3^{rd} Ed., Hanser Verlag, München 1990).

Die genannten Stabilisatorklassen weisen oft spezifische Nachteile auf, die neben der erwünschten Wirkung auftreten. Besonders bezüglich Farbverhalten, Wechselwirkung mit Pigmenten, Verträglichkeit verschiedener, gleichzeitig eingesetzter Stabilisatoren untereinander und mit dem zu stabilisierenden Material, Resistenz gegen Chemikalien und Wasser (Hydrolyseempfindlichkeit), Lagerstabilität, Migrationsverhalten, Verbesserung der Stabilisierung gegen die schädigenden Einflüsse von Wärme und Sauerstoff im Verlauf der Polymerverarbeitung und Verbesserung der Stabilisierung gegen die schädigenden Einflüsse von Licht, Strahlung, Wärme im Langzeitgebrauch besteht ein grosser Bedarf nach neuen Stabilisatoren.

Auf dem Gebiet der Lichtschutzmittel haben sich seit vielen Jahren insbesonders die sterisch gehinderten Amine etabliert, die in den Mechanismus des Polymerabbaus eingreifen. In der jüngeren Vergangenheit wurde ausserdem erkannt, dass diese Verbindungsklasse auch einen gewissen Beitrag zur Langzeitstabilisierung gegen thermisch induzierten Abbau von organischen Polymeren beisteuern (vgl. z. B. P. Gijsman in Polymer Degradation and Stability 43, (1994) 171-174 und F. Gugumus in Polymer Degradation and Stability 44, (1994) 299-322). Im Gegensatz zur Wärmelangzeitstabilisierung von Polymeren wurde eine stabilisierende Wirkung der Verbindungsklasse der sterisch gehinderten Amine im Zusammenhang mit dem thermischen Abbau von organischen Polymeren im Verlauf ihrer Verarbeitung bisher noch nie beobachtet.

Im Zusammenhang mit dem thermisch induzierten Abbau von organischen Polymeren im Verlauf ihrer Verarbeitung wurde in jüngster Vergangenheit erkannt, dass die stabilisierende Wirkung etablierter Stabilisatoren (Antioxidantien auf Basis von sterisch gehinderten Phenolen und Phosphiten/Phosphoniten) durch den Zusatz spezieller Verarbeitungsstabilisatoren deutlich verbessert werden kann. Hier sei insbesonders auf die Verwendung von Lactonen hingewiesen (vgl. u. a. EP-A-842 975, EP-A-839 623). Eine Wirksamkeit von sterisch gehinderten Aminen wurde in diesem Kontext nie beschrieben.

In DE-A-2606026 werden sterisch gehinderte Amine beschrieben, die eine Spirostruktur aufweisen. Die dort beschriebenen Verbindungen werden ausschliesslich als Lichtschutzmittel vorgestellt.

Es wurde nun erstaunlicherweise gefunden, dass eine in DE-A-2 606 026 nicht näher ausgeführte Untergruppe der Formel (1), in der wenigstens einer der zwei Reste X₃ oder X₄ (oder aber beide) einen verzweigten Alkylrest bedeutet, eine aussergewöhnlich hohe Lichtschutzwirkung entfaltet. Die Lichtschutzwirkung der Verbindung (1) ist deutlich grösser als die Wirkung der in DE-A-2606026 beschriebenen Verbindungen (vgl. Experimentalteil). Zusätzlich zeichnen sich die Verbindungen der Formel (1) durch eine Prozess-stabilisierende Wirkung aus, die sie besonders im Verein mit sterisch gehinderten Phenolen und Phosphiten/Phosphoniten in organischen Polymeren entfalten. Diese bei Vertretern der Verbindungsklasse (1) beobachtete Eigenschaft ist für sterisch gehinderte Amine neu und unerwartet.

Gegenstand der Erfindung sind somit Stabilisatoren der allgemeinen Formel (1) 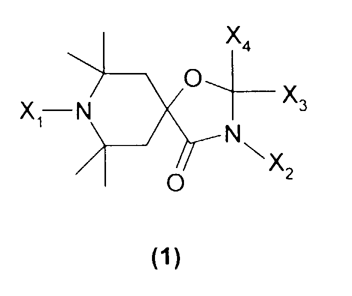 worin
- X₁: Wasserstoff, eine C₁-C₂₂-, vorzugsweise eine C₁-C₅-Alkylgruppe, insbesonders eine Methylgruppe; ein Sauerstoffradikal O*; -OH; -NO; -CH₂CN; Benzyl; Allyl; eine C₁-C₃₀-, vorzugsweise eine C₁-C₁₀-Alkoxygruppe; eine C₅-C₁₂-, vorzugsweise eine C₅-C₆-Cycloalkyloxygruppe; eine C₆-C₁₀-, vorzugsweise eine C₆-C₇-, insbesonders eine C₆-Aryloxygruppe, wobei der Arylrest zusätzlich noch durch eine C₁-C₅-Alkylgruppe oder ein Halogen oder eine Nitrogruppe substituiert sein kann; eine C₇-C₂₀-, vorzugsweise eine C₇-C₁₀-Arylalkyloxygruppe, wobei der Arylrest zusätzlich noch durch eine C₁-C₅-Alkylgruppe oder ein Halogen oder eine Nitrogruppe substituiert sein kann; eine C₃-C₁₀-, vorzugsweise eine C₃-C₆-Alkenylgruppe; eine C₃-C₆-Alkinylgruppe; eine C₁-C₁₀-, vorzugsweise eine C₁-C₅-Acylgruppe; Halogen; einen unsubstituierten oder durch C₁-C₄-, vorzugsweise durch C₁-C₂-Alkyl substituierten Phenylrest, bedeutet;
- X₂: Wasserstoff, einen [CH₂-CH₂-C(O)-O-X₅]-Rest, einen [CH₂-C(CH₃)H-C(O)-O-X₅]-Rest; eine C₁-C₂₂-Alkylgruppe, vorzugsweise eine C₁-C₁₀-, insbesondere C₁-C₄-Alkylgruppe bedeutet;
- X₃ und X₄: unabhängig voneinander Wasserstoff, einen unsubstituierten oder durch C₁-C₄-, vorzugsweise durch C₁-C₂-Alkyl substituierten Phenylrest oder eine C₁- C₂₂-, vorzugsweise eine verzweigte C₄-C₂₂-Alkylgruppe, wobei mindestens einer der zwei Reste X₃ oder X₄ eine verzweigte C₄-C₂₂-Alkylgruppe, vorzugsweise eine verzweigte C₄-C₁₀-, insbesondere eine verzweigte C₄-C₆-Alkylgruppe bedeuten; und worin
- X₅: eine C₁-C₂₂-, vorzugsweise eine C₁₀-C₁₆-, insbesonders eine C₁₂-C₁₄-Alkylgruppe bedeutet.

Die Verbindungen der Formel (1) können erfindungsgemäss auch in vorteilhafter Weise in Mischung mit anderen Stabilisatoren eingesetzt werden, insbesondere in Mischung mit Organophosphiten und/oder Organophosphoniten plus gegebenenfalls sterisch gehinderten Phenolen und/oder weiteren sterisch gehinderten Aminen (HALS). Die Mischungen können zusätzlich auch noch Säurefänger (basische Costabilisatoren) enthalten; in Mischungen, welche sterisch gehinderte Phenole enthalten, können mit Vorteil zusätzlich auch noch schwefelhaltige Costabilisatoren vorhanden sein.

Die Verbindungen der Formel (1) werden zweckmässigerweise in einem Konzentrationsbereich von 0,001-5, vorzugsweise 0,002-0.05 Gew.-%, bezogen auf das zu stabilisierende organische Material, eingesetzt; jede der übrigen Komponenten kann in einem Konzentrationsbereich von 0,001-5, vorzugsweise 0,01-1,0 Gew.-%, vorliegen.

Als sterisch gehinderte Amine kommen für die Zwecke der vorliegenden Erfindung sowohl monomere als auch polymere HALS-Stabilisatoren in Betracht, weiterhin auch Mischungen von polymeren mit monomeren HALS-Stabilisatoren, wie z. B. in EP-A-80 431 und EP-A-632 092 beschrieben.

Die Verbindungen der Formel (1) können besonders vorteilhaft mit monomeren HALS-Stabilisatoren der Formeln A1 bis A10 gemischt werden. 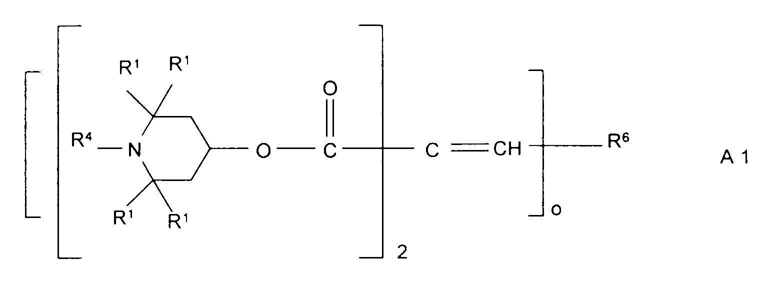 worin
- R¹: Wasserstoff, C₅-C₇-Cycloalkyl oder eine C₁-C₁₂-Alkylgruppe und
- R⁴: entweder Wasserstoff oder eine C₁-C₂₂-Alkylgruppe, ein Sauerstoffradikal O*, -OH, -NO-, CH₂CN, Benzyl, Allyl, eine C₁-C₃₀-Alkoxygruppe, eine C₅-C₁₂-Cycloalkyloxygruppe, eine C₆-C₁₀-Aryloxygruppe, wobei der Arylrest zusätzlich noch substituiert sein kann, eine C₇-C₂₀-Aralkyloxygruppe, wobei der Arylrest zusätzlich noch substituiert sein kann, eine C₃-C₁₀-Alkenylgruppe, eine C₃-C₆-Alkinylgruppe, eine C₁-C₁₀-Acylgruppe, Halogen, unsubstituiertes oder am Phenylring durch C₁-C₄-Alkyl substituiertes C₇-C₉-Phenylalkyl,
- R⁶: einen ein oder mehrfach durch Wasserstoff, C₁-C₄-Alkyl, C₁-C₄-Alkoxy, Halogen, Cyan, Carboxy, Nitro, Amino, C₁-C₄ -Alkylamino, C₁-C₄-Dialkylamino, oder Acyl substituierten aromatischen Rest,
- o: 1 oder 2; bedeutet. 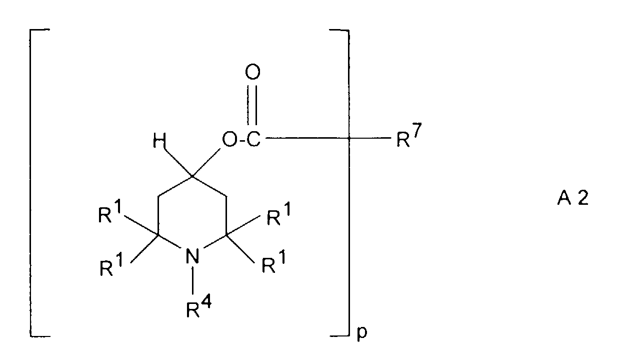
worin
- R¹ und R⁴: die weiter oben angeführten Bedeutungen haben,
- p: 1 oder 2; und
für p = 1 R⁷ C₁-C₂₂-Alkyl, C₂-C₁₈-Oxaalkyl, C₂-C₁₈-Thiaalkyl, C₂-C₁₈-Azaalkyl oder C₂-C₈-Alkenyl,
für p = 2 R⁷ C₁-C₂₂-Alkylen, C₂-C₁₈-Oxaalkylen, C₂-C₁₈-Thiaalkylen, C₂-C₁₈-Azaalkylen oder C₂-C₈-Alkenylen, bedeutet. 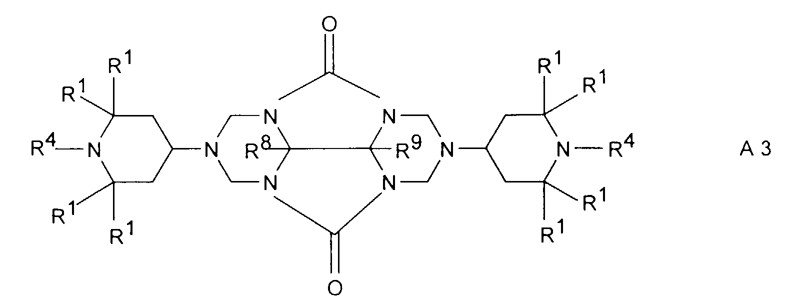
worin
- R¹ und R⁴: die weiter oben angeführten Bedeutungen haben,
- R⁸ und R⁹: unabhängig voneinander Wasserstoff, C₁-C₆-Alkyl, C₇-C₁₂-Aralkyl, -Aryl oder -Carbonsäureester,
- R⁸ und R⁹: zusammen eine Tetra- oder Pentamethylgruppe bedeuten. 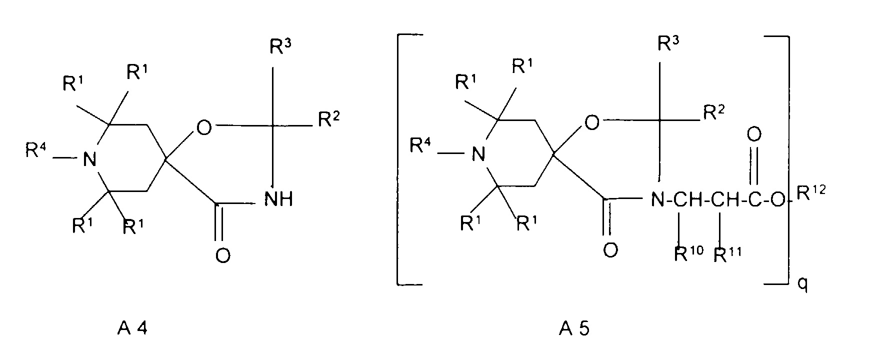
worin
- R¹ und R⁴: die weiter oben angeführten Bedeutungen haben,
- R² und R³: unabhängig voneinander ein Wasserstoffatom, eine C₁-C₁₈ -Alkylgruppe oder zusammen mit dem sie verbindenden C-Atom einen Ring der Ringgrösse 5 bis 13 oder zusammen mit dem sie bindenden C-Atom eine Gruppe der Formel (IV), 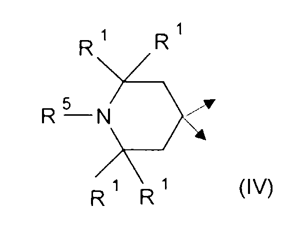
- R⁴ und R⁵: unabhängig voneinander entweder Wasserstoff oder eine C₁-C₂₂ -Alkylgruppe, ein Sauerstoffradikal O*, -OH, -NO, -CH₂CN, Benzyl, Allyl, eine C₁-C₃₀-Alkyloxygruppe, eine C₅-C₁₂-Cycloalkyloxygruppe, eine C₆-C₁₀-Aryloxygruppe, wobei der Arylrest zusätzlich noch substituiert sein kann, eine C₇-C₂₀-Arylalkyloxygruppe, wobei der Arylrest zusätzlich noch substituiert sein kann, eine C₃-C₁₀- Alkenylgruppe, eine C₃-C₆-Alkinylgruppe, eine C₁-C₁₀-Acylgruppe, Halogen, unsubstituiertes oder am Phenylring durch C₁-C₄-Alkyl substituiertes C₇-C₉-Phenylalkyl bedeuten.
- q: eine Zahl von 1 oder 2,
- R¹⁰: Wasserstoff, Methyl, Phenyl oder Carb-C₁-C₂₁-Alkoxy,
- R¹¹: Wasserstoff oder Methyl,
- R¹²: für q = 1 Wasserstoff, C₁-C₂₁-Alkyl, C₂-C₂₂-Alkenyl, C₅-C₁₂-Cycloalkyl, ein Radikal der Formel 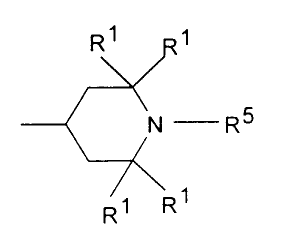 bedeutet, wobei R¹ und R⁵ die weiter oben angeführte Bedeutung haben, und
- R¹²: für q = 2 C₁-C₁₈-Alkylen, C₅-C₉-Cycloalkylen oder Arylen bedeutet. 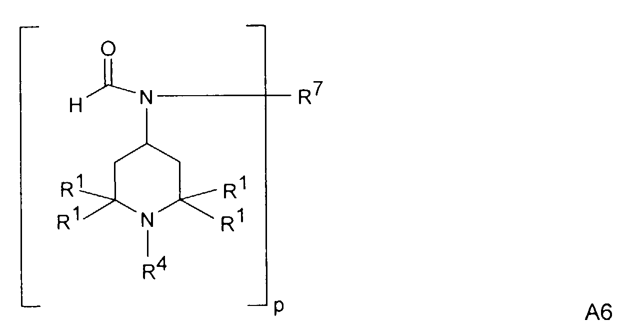
wobei R¹, R⁴, R⁷ und p die oben genannten Definitionen besitzen. 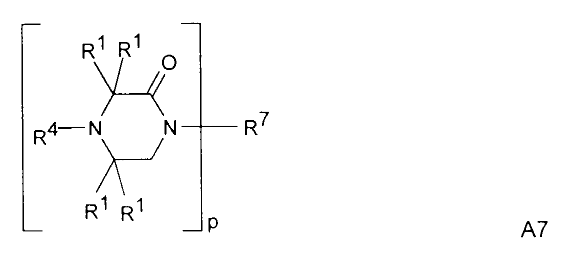 wobei R¹, R⁴, R⁷ und p die oben genannten Definitionen besitzen. 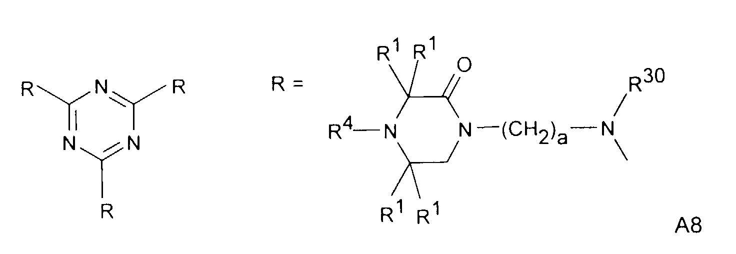 wobei
- R¹, R⁴: die oben genannten Definitionen besitzen,
- R³⁰: Wasserstoff, C₁-C₁₂-Alkyl, C₅-C₁₂-Cycloalkyl, Phenyl oder C₇-C₉-Phenylalkyl, und
- a: eine Zahl von 1 bis 10 bedeutet. 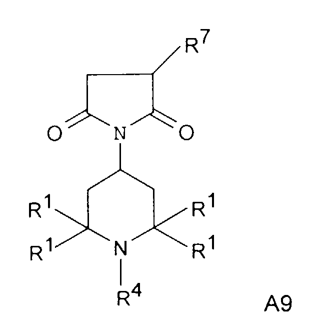
wobei
- R¹ und R⁴: die obige Bedeutung hat und
- R⁷: die in der Formel A2 für p=1 definierte Bedeutung hat.

Ein Produkt A10 erhältlich durch Umsetzung eines Polyamins der Formel A10a mit Formel A10b: 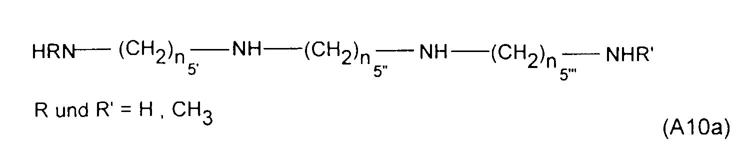 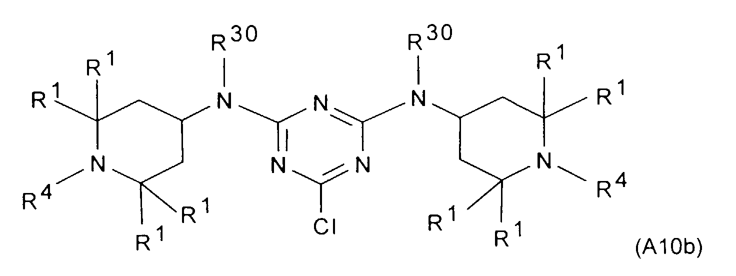 wobei
- R¹, R⁴ und R³⁰: die oben angegebene Bedeutung haben,
- n_{5'}, n_{5"} und n_{5"'}: unabhängig voneinander eine Zahl von 2 bis 12 ist.

Bevorzugt sind Mischungen mit Verbindungen der Formeln A1 bis A10, worin
- R¹: Wasserstoff oder eine C₁-C₄-Alkylgruppe,
- R² und R³: unabhängig voneinander ein Wasserstoffatom, eine C₁-C₈-Alkylgruppe oder zusammen mit dem sie verbindenden C-Atom einen Ring der Ringgrösse 6 bis 12, oder zusammen mit dem sie bindenden C-Atom eine Gruppe der Formel (IV),
- R⁴ und R⁵: unabhängig voneinander entweder Wasserstoff oder eine C₁-C₅-Alkylgruppe, ein Sauerstoffradikal O*, -OH, -NO, -CH₂CN, Benzyl, Allyl, eine C₁-C₁₀-Alkyloxygruppe, eine C₅-C₆-Cycloalkyloxygruppe, eine C₆-C₇-Aryloxygruppe, wobei der Arylrest zusätzlich noch substituiert sein kann; eine C₇-C₁₀-Arylalkyloxygruppe, wobei der Arylrest zusätzlich noch substituiert sein kann, eine C₃-C₆-Alkenylgruppe, eine C₃-C₆-Alkinylgruppe, eine C₁-C₄-Acylgruppe, Halogen, unsubstituiertes oder am Phenylring durch C₁-C₂-Alkyl substituiertes C₇-C₉-Phenylalkyl,
- R⁷: ein geradkettiges C₁-C₁₀-Alkylen (für p = 2); C₁-C₁₂-Alkyl (für p = 1),
- R⁸ und R⁹: unabhängig voneinander Wasserstoff, C₁-C₂-Alkyl, C₇-C₈-Arylalkyl, Aryl- oder Carbonsäureester,
- R¹⁰: Wasserstoff, Methyl, Phenyl oder C₁-C₂-Alkoxy,
- R¹¹: Wasserstoff oder Methyl,
- R¹²: für q = 1 Wasserstoff, C₁-C₁₆-Alkyl, C₂-C₁₆-Alkenyl, C₅-C₆-Cycloalkyl, ein Radikal der Formel 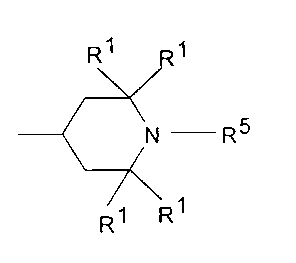
- R¹²: für q = 2 C₁-C₁₆-Alkylen, C₅-C₆-Cycloalkylen oder Arylen bedeutet,
- R³⁰: Wasserstoff, C₁-C₈-Alkyl, C₅-C₇-Cycloalkyl, Phenyl oder C₇-C₈-Phenylalkyl,
- a: 1 bis 5,
- o: 1 und
- p: 2 bis 5 bedeutet.

Ganz besonders bevorzugt sind Mischungen mit Verbindungen der Formeln A1 bis A10, worin
- R¹: Methyl,
- R² und R³: zusammen mit dem sie verbindenden C-Atom einen Ring der Ringgrösse 12, oder zusammen mit dem sie bindenden C-Atom eine Gruppe der Formel (IV),
- R⁴ und R⁵: unabhängig voneinander Wasserstoff, Methyl, Acetyl, Octyloxy oder Cyclohexyloxy,
- R⁶: p-Methoxyphenyl,
- R⁷: Octamethylen, Hexamethylen oder Ethylen (für p = 2), Dodecyl (für p =1),
- R⁸ und R⁹: Wasserstoff,
- R¹⁰: Wasserstoff,
- R¹¹: Wasserstoff,
- R¹²: Dodecamethylen oder Tetradecamethylen,
- R³⁰: Cyclohexyl oder n-Butyl,
- a: gleich 2,
- o: gleich 1,
- p: gleich 2 und
- q: gleich 1 ist.

Ganz besonders eignen sich die folgenden Verbindungen in Mischung mit Verbindungen der Formel (1) 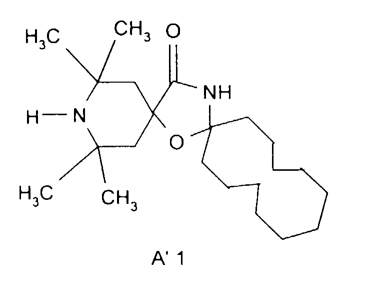 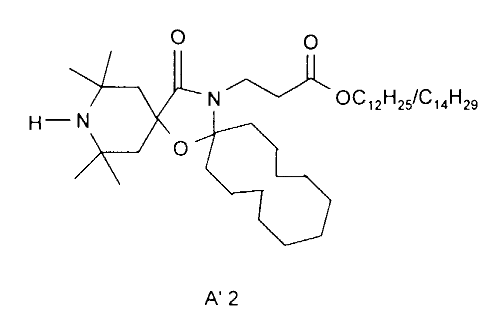 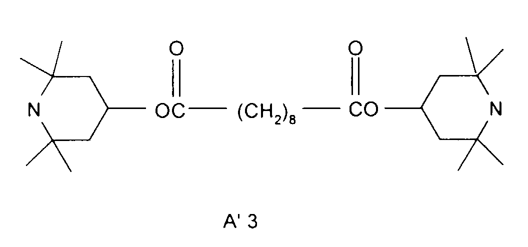 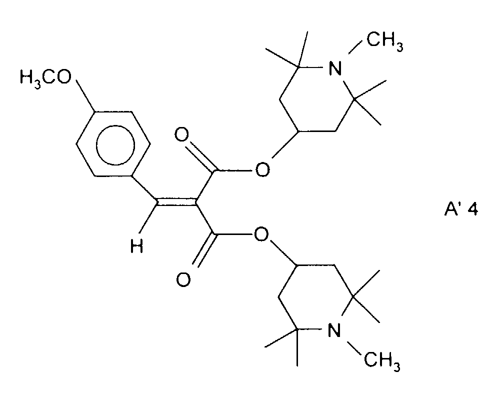 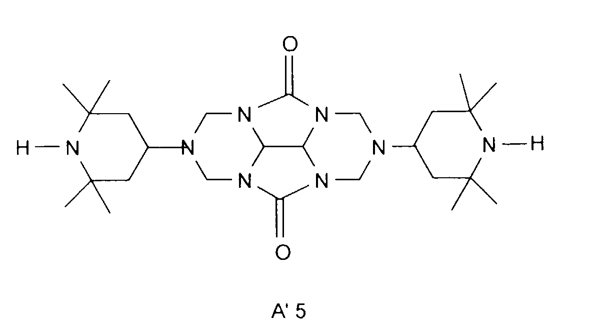 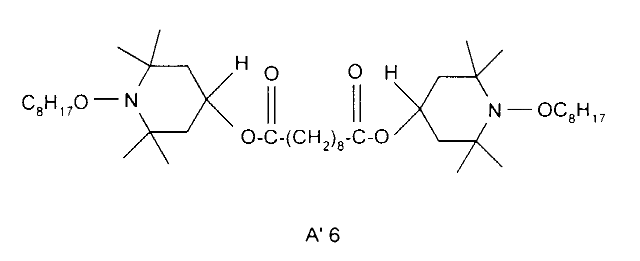 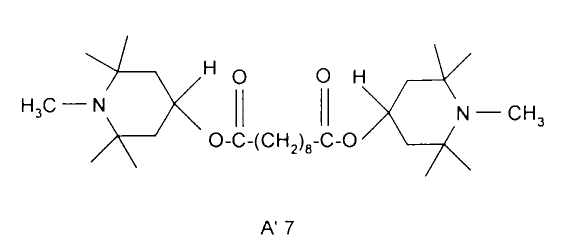 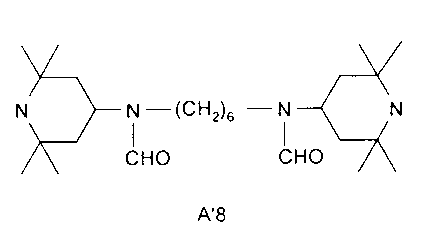 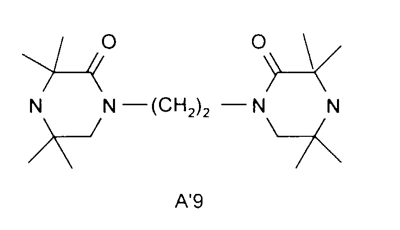 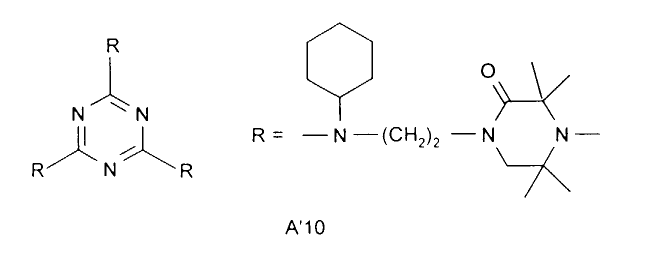 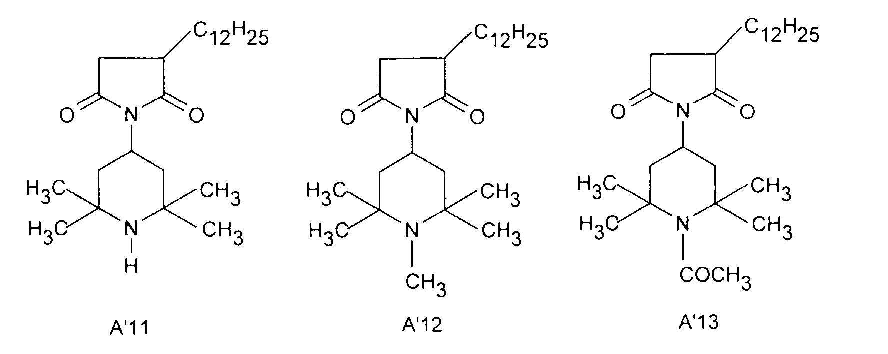 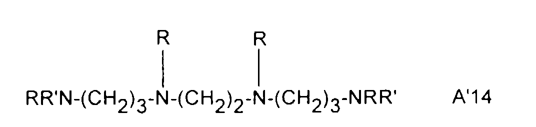 wobei 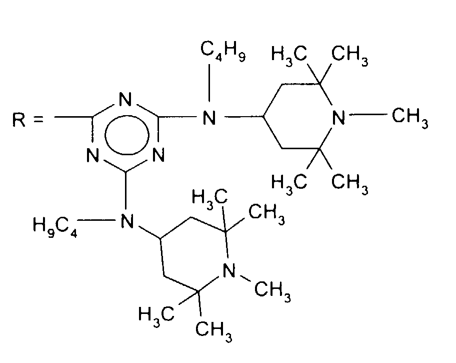 und R'=H, CH₃

In einer besonders geeigneten Ausführungsform der Erfindung verwendet man zur Mischung mit Verbindungen der Formel (1) Stabilisatoren auf Basis sterisch gehinderter Amine wie ®Tinuvin 770, ®Tinuvin 765, ®Tinuvin 123, ®Hostavin N 20, ®Hostavin N 24, ®Uvinul 4049, ®Sanduvor PR 31, ®Uvinul 4050, ®Good-rite UV 3034 oder ®Good-rite 3150, ®Sanduvor 3055, ®Sanduvor 3056, ®Sanduvor 3058, ®Chimassorb 119 und ®Chimassorb 905.

Besonders vorteilhafte polymere HALS-Stabilisatoren zur Mischung mit Verbindungen der Formel (1) sind Verbindungen der Formeln B1 bis B7: 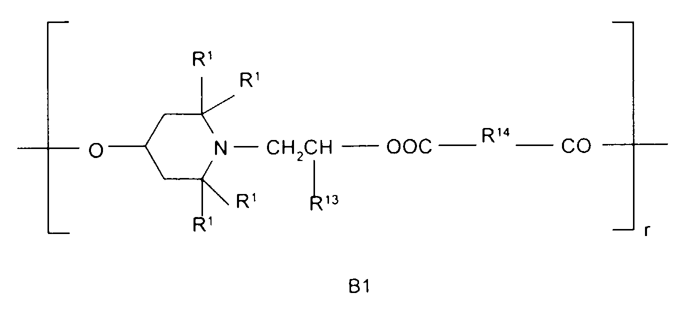 worin
- R¹: Wasserstoff, C₅-C₇-Cycloalkyl oder eine C₁-C₁₂-Alkylgruppe,
- R¹³: Wasserstoff oder Methyl,
- R¹⁴: eine direkte Bindung oder C₁-C₁₀-Alkylen und
- r: eine Zahl von 2 bis 50 bedeutet. 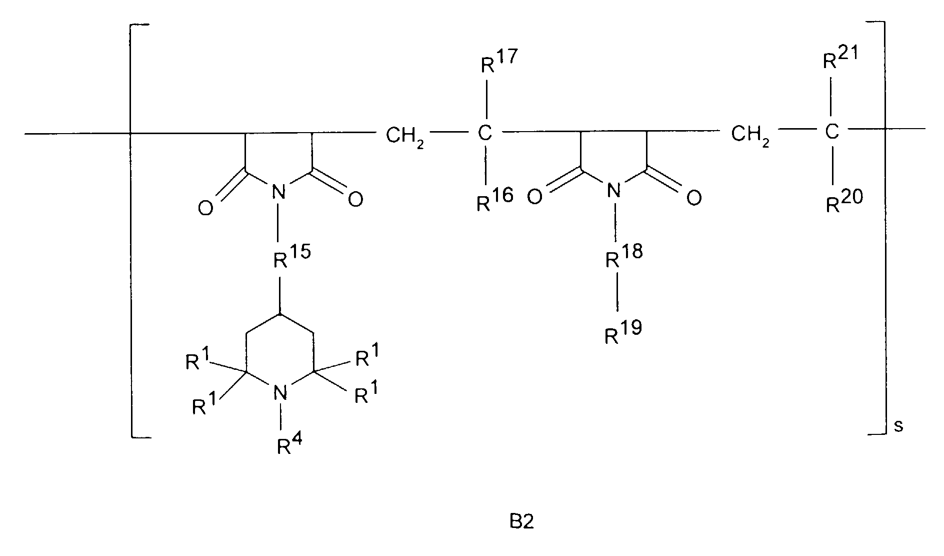
wobei
- R¹ und R⁴: die weiter oben angegebenen Bedeutungen haben,
- R¹⁵ und R¹⁸: unabhängig voneinander eine direkte Bindung oder eine Gruppe -N(R²²)-CO- R²³-CO-N(R²⁴)-,
- R²² und R²⁴: unabhängig voneinander Wasserstoff, C₁-C₈-Alkyl, C₅-C₁₂-Cycloalkyl, Phenyl, C₇-C₉-Phenylalkyl, oder eine Gruppe der Formel

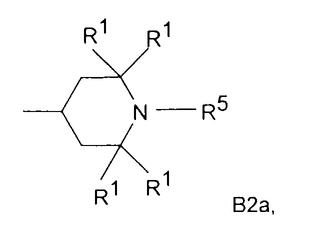

- R²³: eine direkte Bindung oder C₁-C₄-Alkylen,
- R¹⁶, R¹⁷, R²⁰, R²¹: unabhängig voneinander Wasserstoff, C₁-C₃₀-Alkyl, C₅-C₁₂-Cycloalkyl, Phenyl, oder eine Gruppe der Formel B2a,
- R¹⁹: Wasserstoff, C₁-C₃₀-Alkyl, C₅-C₁₂-Cycloalkyl, C₇-C₉-Phenylalkyl, Phenyl oder eine Gruppe der Formel B2a bedeuten und
- s: eine Zahl von 1 bis 50 ist. 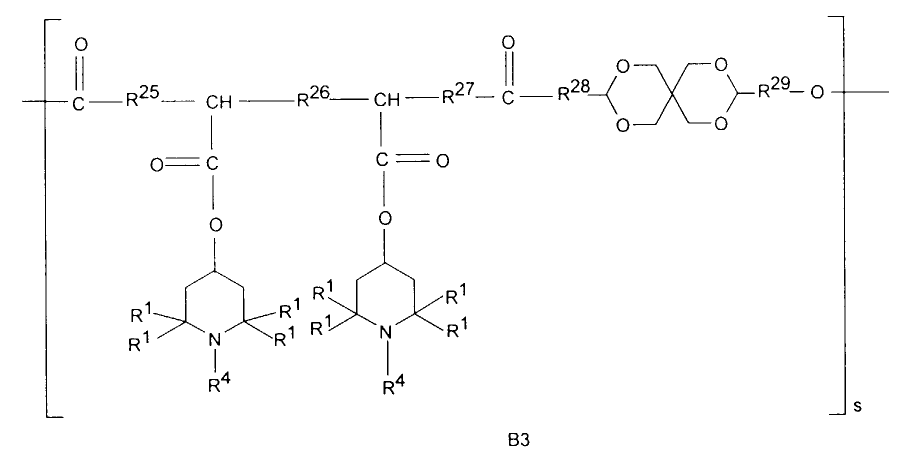
wobei
- R¹, R⁴ und s: die oben angegebenen Bedeutungen haben,
- R²⁵, R²⁶, R²⁷, R²⁸ und R²⁹: unabhängig voneinander eine direkte Bindung oder C₁-C₁₀-Alkylen sind.

Ein Produkt B4 erhältlich durch Umsetzung eines durch Reaktion eines Polyamins der Formel B4a mit Cyanursäurechlorid erhaltenen Produktes mit einer Verbindung der Formel B4b, 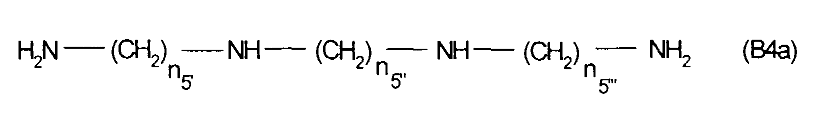 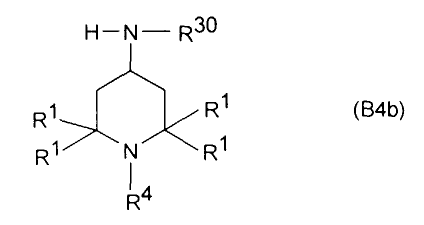 wobei
- R¹ und R⁴: die weiter oben angegebenen Bedeutungen haben,
- n_{5'}, n_{5"} und n_{5"'}: unabhängig voneinander eine Zahl von 2 bis 12 sind,
- R³⁰: die oben angegebene Bedeutung hat; wobei B4 eine Verbindung der Formel B4-1, B4-2, B4-3 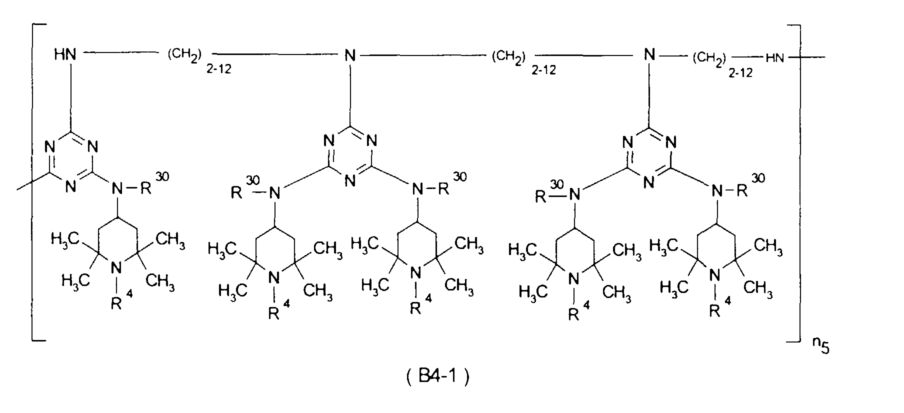 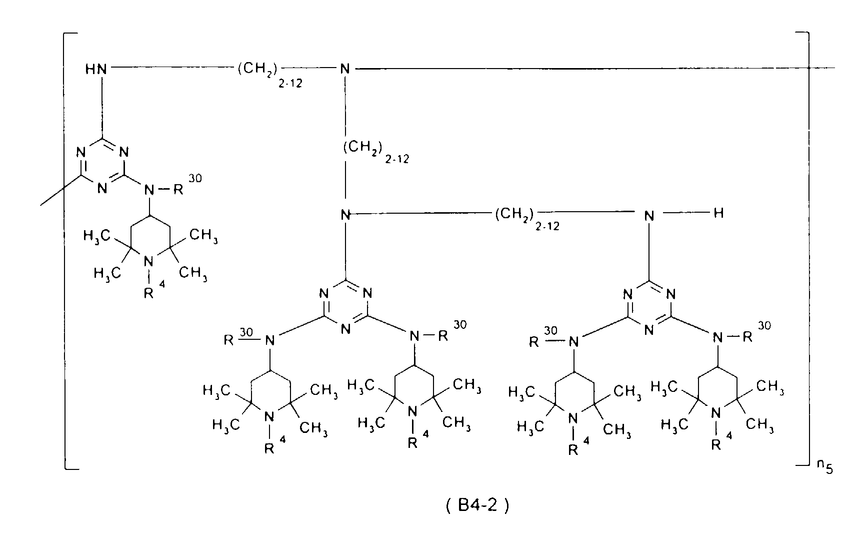 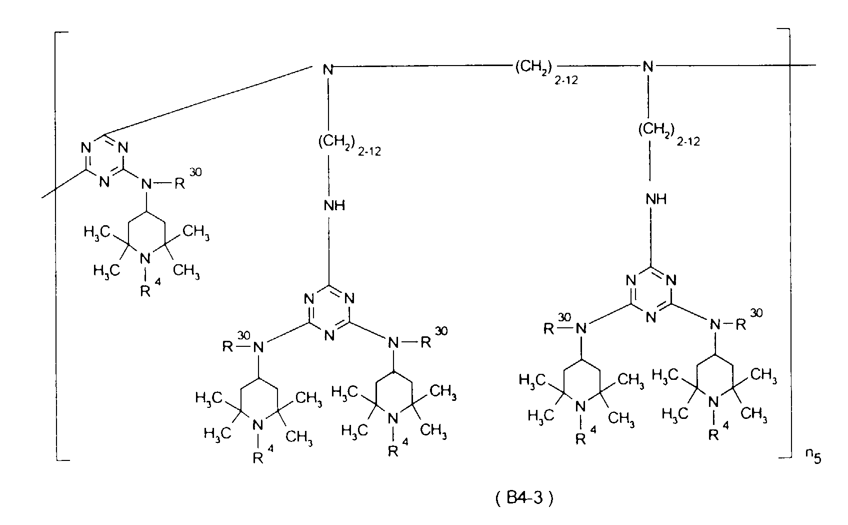
oder ein Gemisch daraus darstellt, worin
- n₅: 1 bis 20,
- R⁴ und R³⁰: die oben angegebene Bedeutung haben. 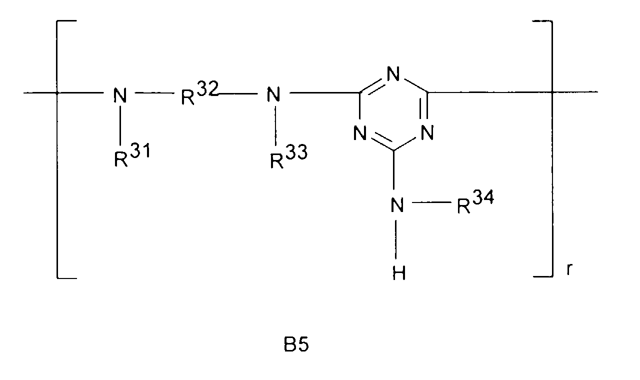
wobei
- r: die bei Formel B1 angegebene Bedeutung hat,
- R³¹, R³³ und R³⁴: unabhängig voneinander Wasserstoff, C₁-C₁₂-Alkyl, C₅-C₁₂-Cycloalkyl, durch C₁-C₄-Alkyl substituiertes C₅-C₁₂-Cycloalkyl, Phenyl, durch -OH und/oder C₁-C₁₀-Alkyl substituiertes Phenyl, C₇-C₉-Phenylalkyl, am Phenylrest durch -OH und/oder C₁-C₁₀-Alkyl substituiertes C₇-C₉-Phenylkalkyl oder eine Gruppe der Formel B5a bedeuten, 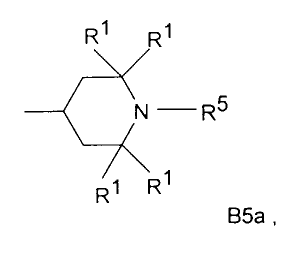 wobei R¹ und R⁵ die oben angegebenen Bedeutungen haben, und
- R³²: C₂-C₁₈-Alkylen, C₅-C₇-Cycloalkylen oder C₁-C₄-Alkylendi(C₅-C₇-Cyloalkylen) ist, oder
- die Reste R³¹, R³² und R³³: zusammen mit den Stickstoffatomen, an die sie gebunden sind, einen 5- bis 10-gliedrigen heterocyclischen Ring bilden, und wobei mindestens einer der Reste R³¹, R³³ und R³⁴ eine Gruppe der Formel B5a darstellt. 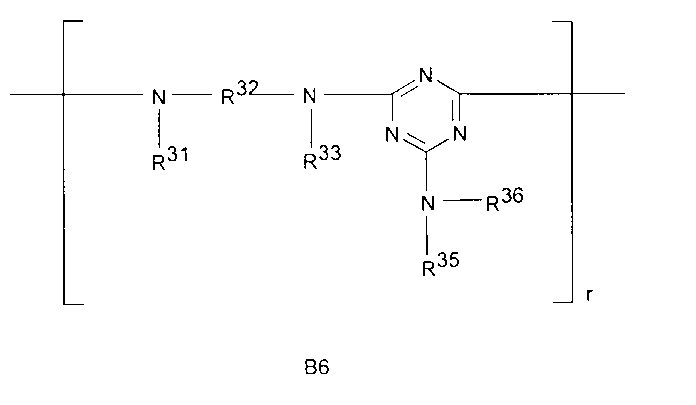
worin
- R³¹, R³², R³³ und r: die oben angegebenen Bedeutungen haben,
- R³⁵ und R³⁶: unabhängig voneinander die Definition von R³⁴ haben kann, oder
- R³⁵ und R³⁶: zusammen mit dem Stickstoffatom, an das sie gebunden sind, einen 5- bis 10-gliedrigen heterozyklischen Ring bilden, wobei dieser zusätzlich zum Stickstoff-Heteroatom noch ein oder mehrere Heteroatome, vorzugsweise ein Sauerstoffatom, enthalten kann und mindestens einer der Reste R³¹, R³³, R³⁵ und/oder R³⁶ eine Gruppe der Formel (B5a) darstellt. 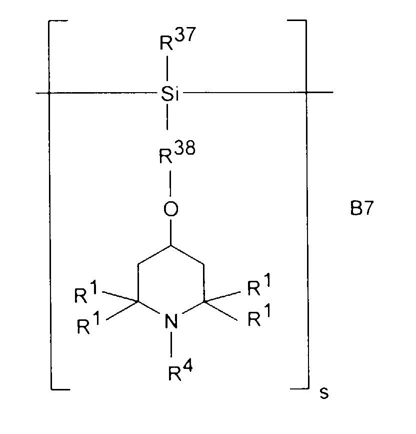
wobei
- R¹ und R⁴: die weiter oben angegebene Bedeutung haben,
- s: die in Formel B3 angegebenen Bedeutung hat,
- R³⁷: C₁-C₁₀-Alkyl, C₅-C₁₂-Cycloalkyl, durch C₁-C₄-Alkyl substituiertes C₅-C₁₂-Cycloalkyl, Phenyl oder durch C₁-C₁₀-Alkyl substituiertes Phenyl ist, und
- R³⁸: C₃-C₁₀-Alkylen darstellt.

Die als Komponenten B1 bis B4 beschriebenen Verbindungen sind im wesentlichen bekannt (teilweise im Handel erhältlich) und können nach bekannten Verfahren, zum Beispiel wie in US 4,233,412, US 4,340,534, US 4,857,595, DD-A-262 439 (Derwent 89-122 983/17, Chemical Abstracts 111:58 964u), DE-A-4 239 437 (Derwent 94-177 274/22), US 4,529,760, US 4,477,615 und Chemical Abstracts - CAS No. 136 504-96-6 beschrieben, hergestellt werden.

Die Komponente B4 kann in Analogie zu bekannten Verfahren zum Beispiel durch Umsetzung von einem Polyamin der Formel B4a mit Cyanursäurechlorid in einem molaren Verhältnis von 1:2 bis 1:4 in Gegenwart von wasserfreiem Lithium-, Natrium- oder Kaliumcarbonat in einem organischen Lösungsmittel wie 1,2-Dichlorethan, Toluol, Xylol, Benzol, Dioxan oder tert-Amylalkohol bei einer Temperatur von -20°C bis +10°C, bevorzugt -10°C bis +10°C, insbesondere 0°C bis +10°C, während 2 bis 8 Stunden und anschliessender Reaktion des erhaltenen Produktes mit einem 2,2,6,6-Tetramethyl-4-piperidylamin der Formel B4b hergestellt werden. Das molare Verhältnis von 2,2,6,6-Tetramethyl-4-piperidylamin zu eingesetztem Polyamin der Formel B4a beträgt beispielsweise 4:1 bis 8:1. Die Menge an 2,2,6,6-Tetramethyl-4-piperidylamin kann auf einmal oder in mehreren Portionen im Abstand von einigen Stunden zugegeben werden.

Bevorzugt beträgt das Verhältnis Polyamin der Formel B4a: Cyanursäurechlorid: 2,2,6,6-Tetramethyl-4-piperidylamin der Formel B4b 1:3:5 bis 1:3:6.

Allgemein kann die Komponente B4 zum Beispiel durch eine Verbindung der Formel B4-1, B4-2 oder B4-3 wiedergegeben werden. Sie kann auch als Gemisch dieser drei Verbindungen vorliegen.

Eine bevorzugte Bedeutung der Formel B4-1 ist

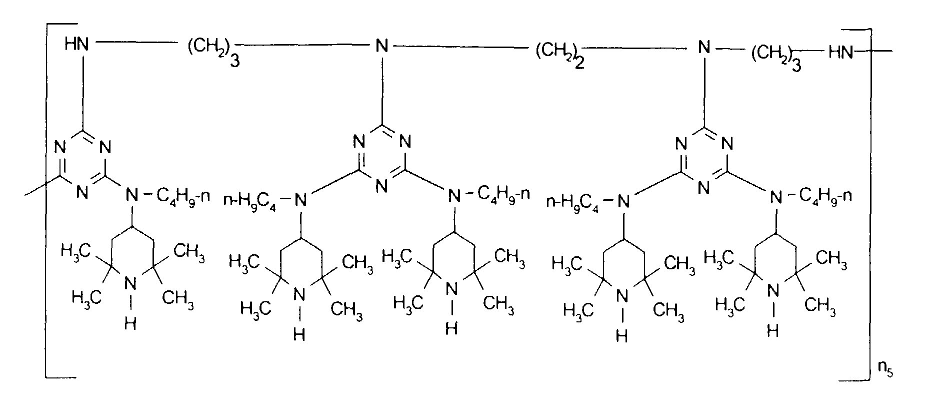

Eine bevorzugte Bedeutung der Formel B4-2 ist

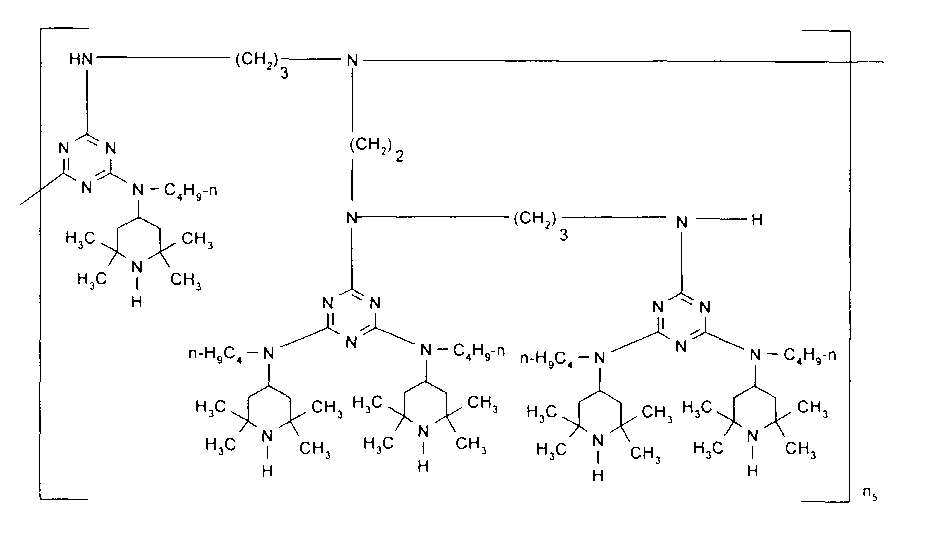

Eine bevorzugte Bedeutung der Formel B4-3 ist

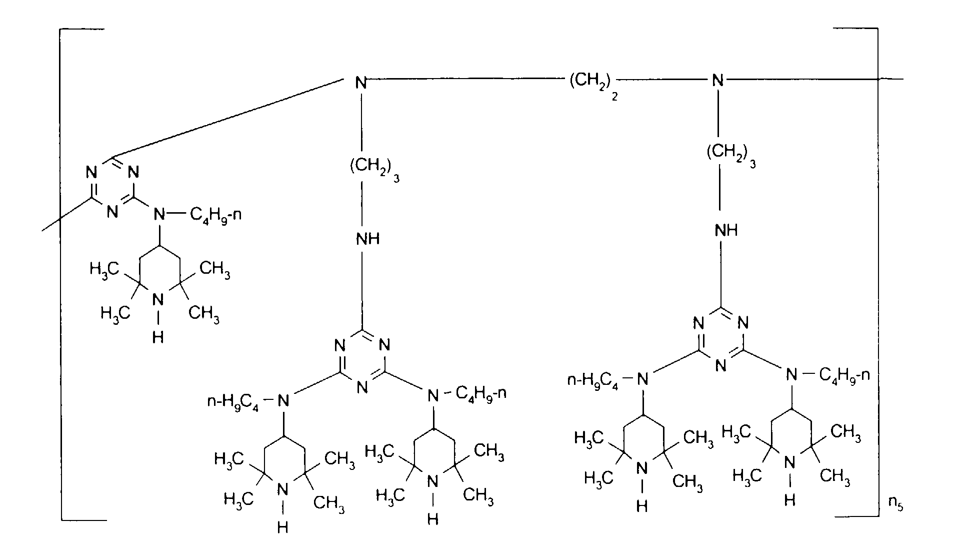

Bevorzugt sind zur Mischung mit Verbindungen der Formel (1) Verbindungen der Formeln B1 bis B7, worin
- R¹: Wasserstoff oder eine C₁-C₄-Alkylgruppe,
- R² und R³: unabhängig voneinander ein Wasserstoffatom, eine C₁-C₈-Alkylgruppe oder zusammen mit dem sie verbindenden C-Atom einen Ring der Ringgrösse 6 bis 12, oder zusammen mit dem sie bindenden C-Atom eine Gruppe der Formel (IV),
- R⁴ und R⁵: unabhängig voneinander entweder Wasserstoff oder eine C₁-C₅-Alkylgruppe, ein Sauerstoffradikal O*, -OH, -NO, -CH₂CN, Benzyl, Allyl, eine C₁-C₁₀-Alkyloxygruppe, eine C₅-C₆-Cycloalkyloxygruppe, eine C₆-C₇-Aryloxygruppe, wobei der Arylrest zusätzlich noch substituiert sein kann, eine C₇-C₁₀-Arylalkyloxygruppe, wobei der Arylrest zusätzlich noch substituiert sein kann, eine C₃-C₆-Alkenylgruppe, eine C₃-C₆-Alkinylgruppe, eine C₁-C₄-Acylgruppe, Halogen, unsubstituiertes oder am Phenylring durch C₁-C₂-Alkyl substituiertes C₇-C₉-Phenylalkyl bedeuten,
- R¹³: Wasserstoff oder Methyl,
- R¹⁴: C₁-C₅-Alkylen,
- R¹⁷, R²¹: Wasserstoff oder C₁-C₄-Alkyl,
- R¹⁵, R¹⁸: eine direkte Bindung,
- R¹⁶, R²⁰: C₁-C₂₅-Alkyl, Phenyl,
- R¹⁹: Wasserstoff, C₁-C₁₂-Alkyl oder eine Gruppe der Formel B2a
- R²⁵, R²⁶, R²⁷, R²⁸ und R²⁹: unabhängig voneinander eine direkte Bindung oder C₁-C₅-Alkylen,
- R³⁰: Wasserstoff, C₁-C₄-Alkyl, C₅-C₆-Cycloalkyl, Phenyl,
- R³¹, R³³ und R³⁴: unabhängig voneinander Wasserstoff, C₁-C₁₀-Alkyl, C₅-C₆-Cycloalkyl oder eine Gruppe der Formel B5a bedeutet,
- R³²: C₂-C₁₀-Alkylen, C₅-C₆-Cycloalkylen,
- R³⁵ und R³⁶: unabhängig voneinander die Definition von R³⁴, oder R³⁵ und R³⁶ zusammen mit dem N-Atom, an das sie gebunden sind einen 5 bis 7-gliedrigen heterocyclischen Ring bilden, wobei dieser noch ein oder mehrere Heteroatome, vorzugsweise ein O-Atom enthalten kann und mindestens einer der Reste R³¹,R³³,R³⁵ und/oder R³⁶ eine Gruppe der Formel B5a darstellt,
- R³⁷: C₁-C₅-Alkyl, C₅-C₆-Cycloalkyl oder Phenyl,
- R³⁸: C₃-C₅-Alkylen und
- n_{5'},n_{5"},n_{5"'}: 2 bis 4 bedeuten.

Ganz besonders bevorzugt zur Mischung mit Verbindungen der Formel (1) sind Verbindungen der Formeln B1 bis B7, worin
- R¹: Methyl,
- R² und R³: zusammen mit dem sie verbindenden C-Atom einen Ring der Ringgrösse 12, oder zusammen mit dem sie bindenden C-Atom eine Gruppe der Formel (IV),
- R⁴ und R⁵: unabhängig voneinander Wasserstoff, Acetyl, Methyl, Octyloxy oder Cyclohexyloxy,
- R¹³: Wasserstoff,
- R¹⁴: Ethylen,
- R¹⁷, R²¹: Wasserstoff oder Methyl,
- R¹⁵, R¹⁸: eine direkte Bindung,
- R¹⁶, R²⁰: C₁-C₂₅-Alkyl, Phenyl,
- R¹⁹: Hexadecyl oder eine Gruppe der Formel B2a,
- R²⁵, R²⁷: Methylen,
- R²⁶: eine direkte Bindung,
- R²⁸: 2,2-Dimethylethylen,
- R²⁹: 1,1-Dimethylethylen,
- R³⁰: n-Butyl,
- R³¹, R³³ und R³⁴: unabhängig voneinander Isooctyl, Cyclohexyl oder 2,2,6,6-Tetramethylpiperid-4-yl bedeuten, wobei mindestens einer der Reste R³¹, R³³ und R³⁴ 2,2,6,6-Tetramethylpiperid-4-yl bedeuten muss,
- R³²: Hexamethylen,
- R³⁵ und R³⁶: unabhängig voneinander die Definition von R³⁴, oder R³⁵ und R³⁶ zusammen mit dem N-Atom, an das sie gebunden sind einen 6-gliedrigen heterocyclischen Ring bilden, wobei dieser noch ein Sauerstoffatom enthält und folglich Morpholin ist, wobei mindestens einer der Reste R³¹,R³³,R³⁵ und/oder R³⁶ einen Rest 2,2,6,6-Tetramethylpiperid-4-yl bedeuten muss,
- R³⁷: Methyl,
- R³⁸: Trimethylen,
- n_{5'},n_{5"},n_{5"'}: 2 bis 4 bedeuten.

Ganz besonders bevorzugt handelt es sich zur Mischung mit Verbindungen der Formel (1) bei den polymeren HALS-Verbindungen um die folgenden Substanzen: 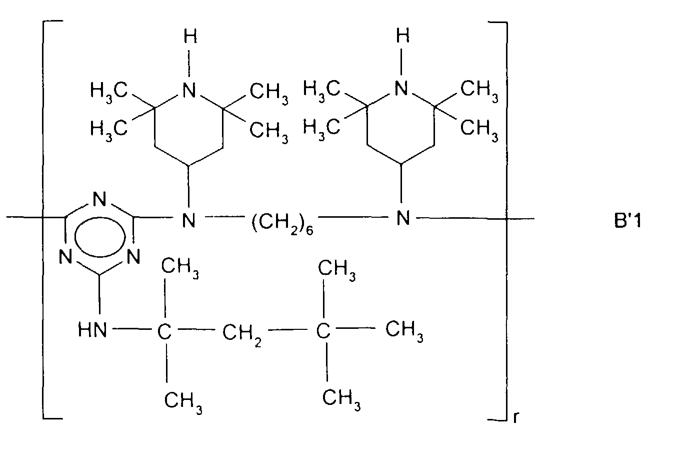 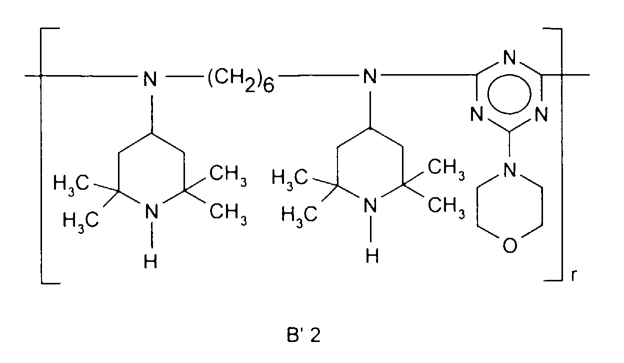 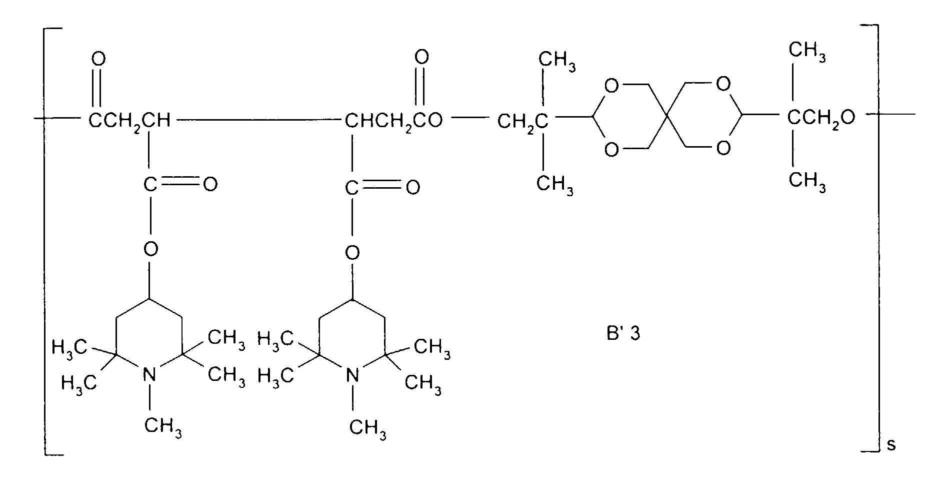 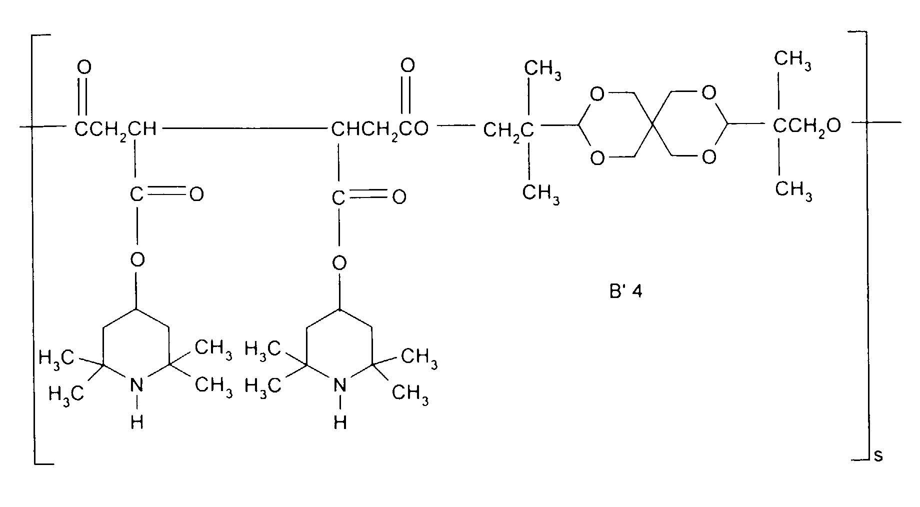 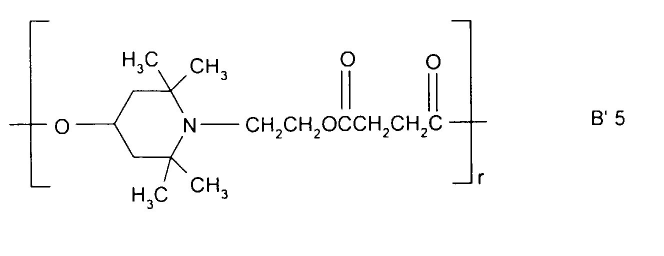 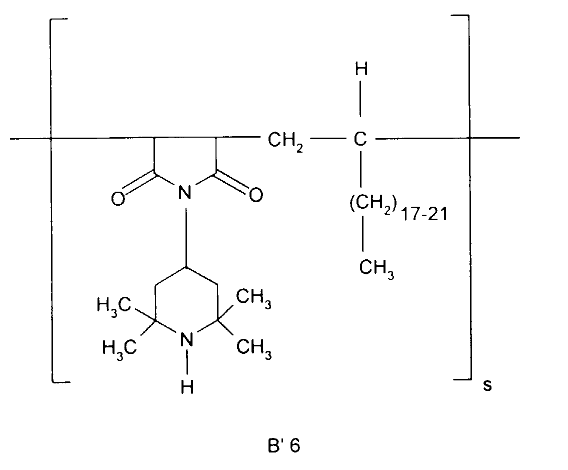 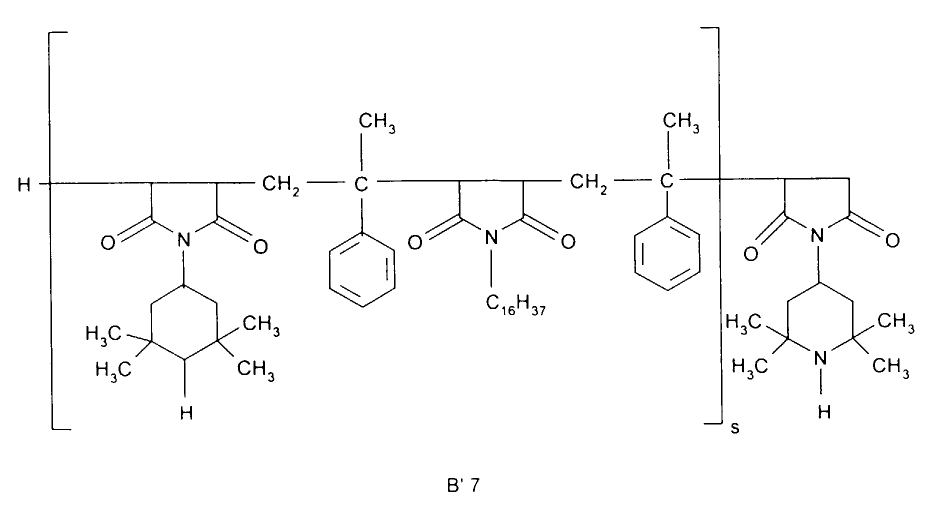 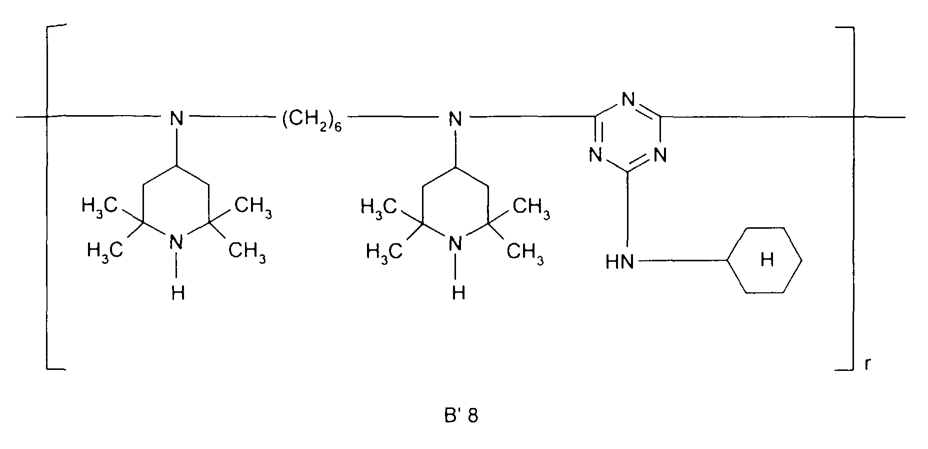 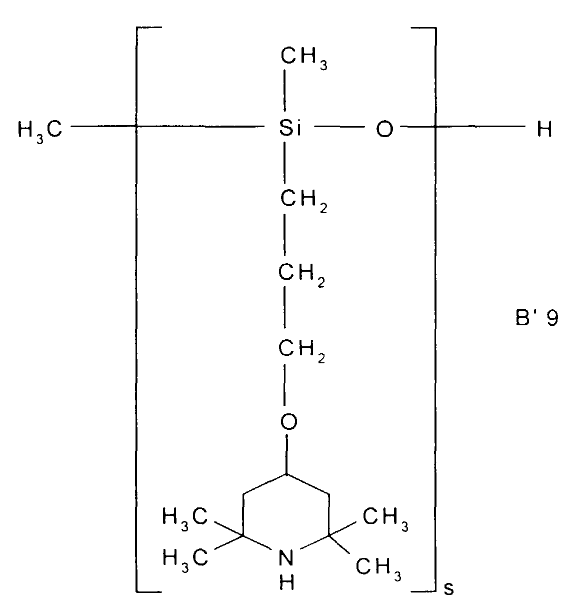 ein Produkt B'10 erhältlich durch Umsetzung eines durch Reaktion eines Polyamins der Formel B'10a:

H₂N-(CH₂)₃-NH-(CH₂)₂-NH-(CH₂)₃-NH₂ (B'10a)

mit Cyanursäurechlorid erhaltenen Produktes mit einer Verbindung der Formel (B'10b) 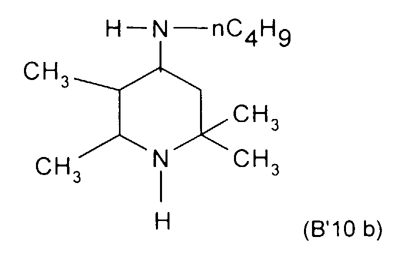 wobei B'10 eine Verbindung der Formel B4-1', B4-2', B4-3' 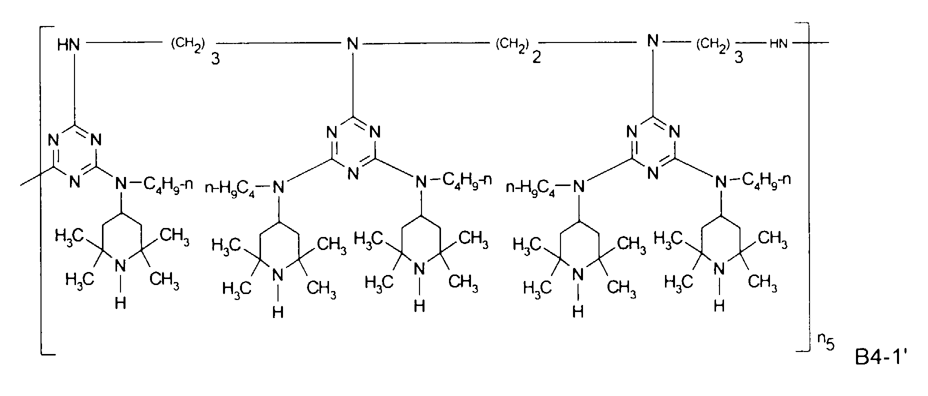 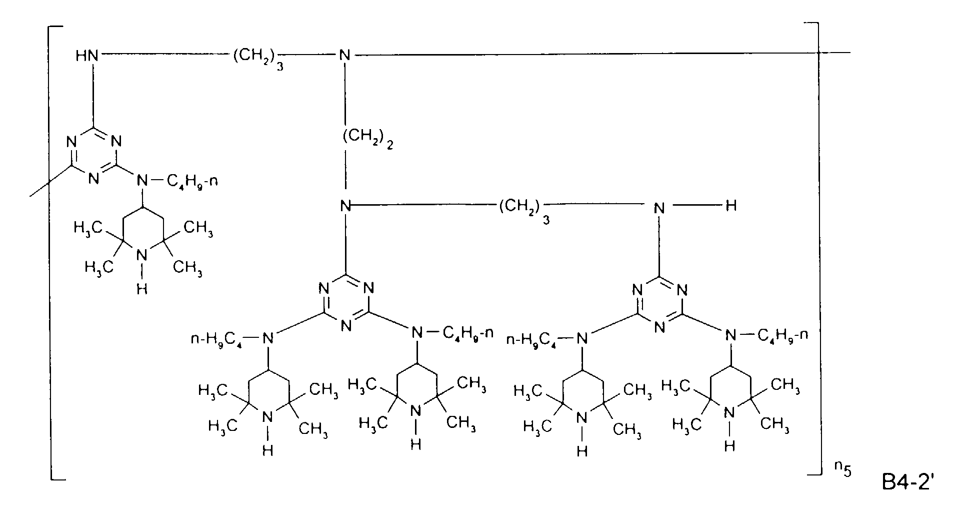 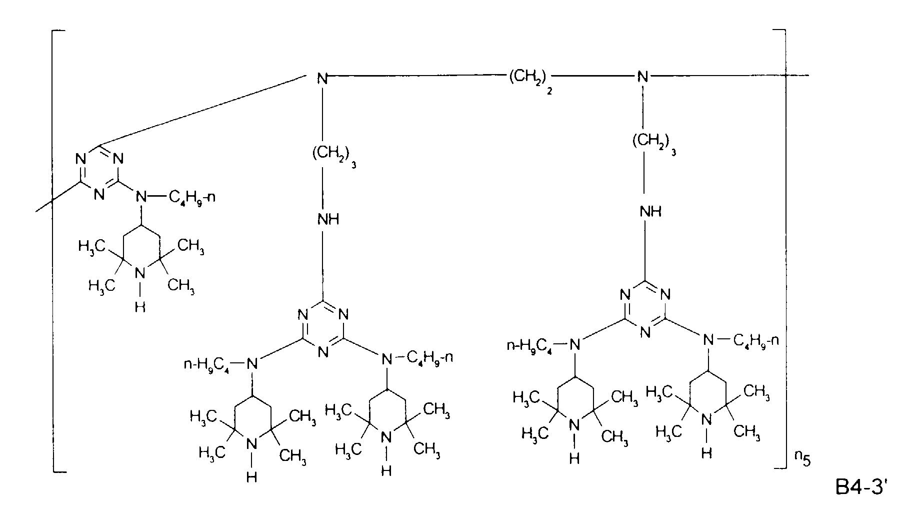 oder ein Gemisch daraus bedeutet, wobei n₅ 1 bis 20 bedeutet.

Besonders bevorzugt zur Mischung mit Verbindungen der Formel (1) sind ®Chimassorb 944, ®Tinuvin 622, ®Dastib 1082, ®Uvasorb HA 88, ®Uvinul 5050, ®Lowilite 62, ®Uvasil 299, ®Cyasorb 3346, ®MARK LA 63, ®MARK LA 68 oder ®Luchem B 18.

Geeignet zur Mischung mit Verbindungen der Formel (1) sind auch Kombinationen polymerer HALS-Stabilisatoren, wie z. B. beschrieben in EP-A-252 877, EP-A-709 426, Research Disclosure Jan. 1993, Nr. 34 549 und EP-A-723 990.

Als Organophosphite bzw. Organophosphonite eignen sich zur Mischung mit Verbindungen der Formel (1) Verbindungen der Formeln C1 bis C7: 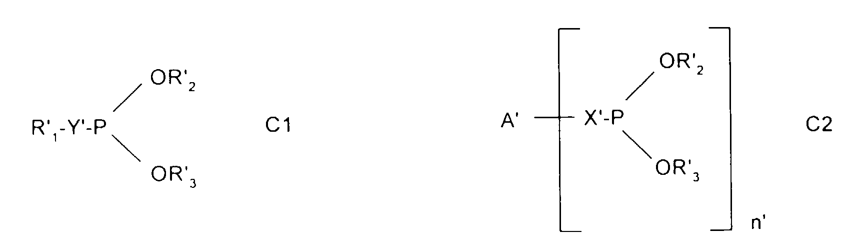 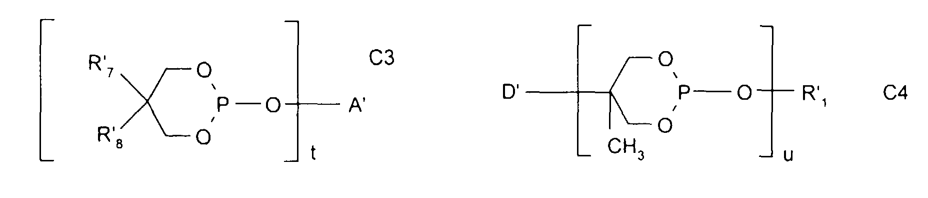 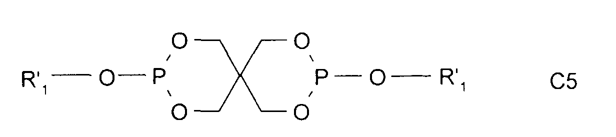 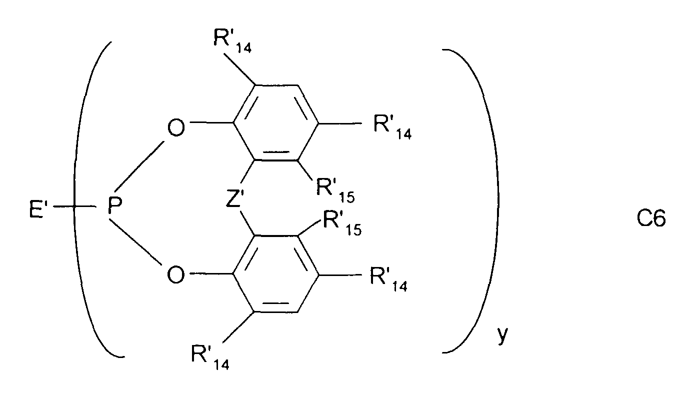 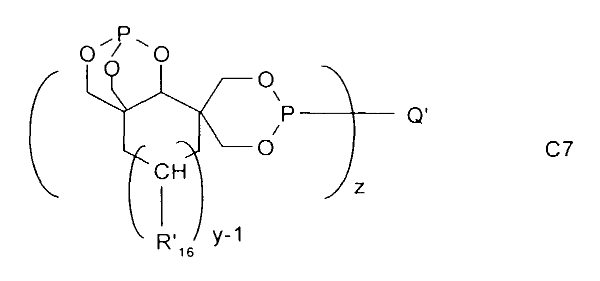 worin die Indices ganzzahlig sind und n' für 2, 3 oder 4; u für 1 oder 2; t für 2 oder 3; y für 1, 2 oder 3; und z für 1 bis 6 steht; A', wenn n' 2 ist, Alkylen mit 2 bis 18 Kohlenstoffatomen; durch -S-, -O- oder -NR'₄- unterbrochenes Alkylen mit 2 bis 12 Kohlenstoffatomen; ein Rest einer der Formeln 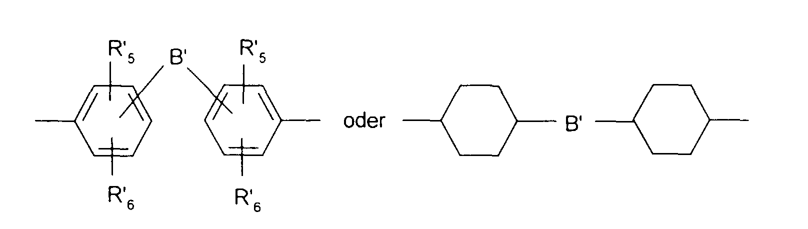 oder Phenylen ist;
- A': wenn n' 3 ist, ein Rest der Formel -CᵣH₂ᵣ₋₁ ist;
- A': wenn n'4 ist, den Rest der Formel C(CH₂)₄- bedeutet;
- A": die Bedeutung von A', wenn n' 2 ist, hat;
- B': einen Rest der Formel -CH₂-; -CHR'₄-; -CR'₁R'₄-; -S- oder eine direkte Bindung darstellt; oder C₅-C₇-Cycloalkyliden; oder mit 1 bis 4 C₁-C₄-Alkylresten in Position 3, 4 und/oder 5 substituierters Cyclohexyliden bedeutet,
- D': wenn u 1 ist, Methyl und, wenn u 2 ist, -CH₂OCH₂- bedeutet;
- E': wenn y 1 ist, Alkyl mit 1 bis 18 Kohlenstoffatomen, Phenyl, ein Rest der Formel -OR'₁ oder Halogen ist;
- E': wenn y 2 ist, ein Rest der Formel -O-A"-O- ist;
- E': wenn y 3 ist, ein Rest der Formel R'₄C(CH₂O)₃- oder N(CH₂-CH₂-O-)₃ ist;
- Q': für den Rest eines mindestens z-wertigen Alkohols oder Phenols steht, wobei dieser über das (die) alkoholische(n) bzw. phenolische(n) O-Atom(e) an das (die) P-Atom(e) gebunden ist;
- R'₁, R'₂ und R'₃: unabhängig voneinander Alkyl mit 1 bis 30 Kohlenstoffatomen; mit Halogen, -COOR₄', -CN oder -CONR₄'R₄' substituiertes Alkyl mit 1 bis 18 Kohlenstoffatomen; durch -S-, -O- oder -NR'₄- unterbrochenes Alkyl mit 2 bis 18 Kohlenstoffatomen; Phenyl-C₁-C₄-alkyl; Cycloalkyl mit 5 bis 12 Kohlenstoffatomen; Phenyl oder Naphthyl; mit Halogen, 1 bis 3 Alkylresten oder Alkoxyresten mit insgesamt 1 bis 18 Kohlenstoffatomen oder mit Phenyl-C₁-C₄-Alkyl substituiertes Phenyl oder Naphthyl; oder ein Rest der Formel 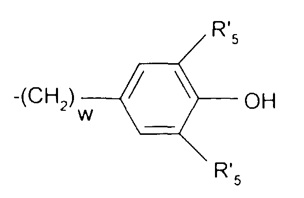 sind, worin w eine ganze Zahl aus dem Bereich 3 bis 6 bedeutet;
- R'4: beziehungsweise die Reste R'₄ unabhängig voneinander Wasserstoff, Alkyl mit 1 bis 18 Kohlenstoffatomen, Cycloalkyl mit 5 bis 12 Kohlenstoffatomen, oder Phenylalkyl mit 1 bis 4 Kohlenstoffatomen im Alkylteil;
- R'₅ und R'₆: unabhängig voneinander Wasserstoff, Alkyl mit 1 bis 8, Kohlenstoffatomen oder Cycloalkyl mit 5 oder 6 Kohlenstoffatomen sind;
- R'₇ und R'₈: im Fall t = 2 unabhängig voneinander C₁-C₄-Alkyl oder zusammen einen 2,3-Dehydropentamethylenrest darstellen; und
- R'₇ und R'₈: im Fall t = 3, Methyl bedeuten;
- die Substituenten R'14: unabhängig voneinander Wasserstoff, Alkyl mit 1 bis 9 Kohlenstoffatomen oder Cyclohexyl sind;
- die Substituenten R'₁₅: unabhängig voneinander Wasserstoff oder Methyl; und
- R'₁₆: Wasserstoff oder C₁-C₄-Alkyl darstellt und im Fall, dass mehrere Reste R'₁₆ vorhanden sind, die Reste R'₁₆ gleich oder verschieden sind;
- X' und Y': jeweils eine direkte Bindung oder -O- darstellen; und
- z: eine direkte Bindung; -CH₂-; -C(R'₁₆)₂- oder -S- ist.

Besonders bevorzugt zur Mischung mit Verbindungen der Formel (1) sind Phosphite oder Phosphonite der Formeln C1, C2, C5 oder C6, worin
- n': für die Zahl 2 und y für die Zahl 1 oder 2 steht;
- A': Alkylen mit 2 bis 18 Kohlenstoffatomen; p-Phenylen oder p-Biphenylen ist;
- E': im Fall y = 1 C₁-C₁₈-Alkyl, -OR₁ oder Fluor; und im Fall y = 2 p-Biphenylen ist;
- R'₁, R'₂ und R'₃: unabhängig voneinander Alkyl mit 1 bis 18 Kohlenstoffatomen; Phenyl-C₁-C₄-Alkyl; Cyclohexyl; Phenyl; mit 1 bis 3 Alkylresten mit insgesamt 1 bis 18 Kohlenstoffatomen substituiertes Phenyl bedeutet;
- die Substituenten R'₁₄: unabhängig voneinander Wasserstoff oder Alkyl mit 1 bis 9 Kohlenstoffatomen sind;
- R'₁₅: Wasserstoff oder Methyl ist;
- X': eine direkte Bindung;
- Y': -O-; und
- Z': eine direkte Bindung oder -CH(R'₁₆)- ist.

Ganz besonders bevorzugt zur Mischung mit Verbindungen der Formel (1) sind Phosphite oder Phosphonite einer der Formeln C1, C2, C5 oder C6 worin
- n': für die Zahl 2 und y für die Zahl 1 steht;
- A': p-Biphenylen ist;
- E': C₁-C₁₈-Alkoxy ist;
- R'₁, R'₂ und R'₃: unabhängig voneinander mit 2 oder 3 Alkylresten mit insgesamt 2 bis 12 Kohlenstoffatomen substitutiertes Phenyl bedeutet;
- die Substituenten R'₁₄: unabhängig voneinander Methyl oder tert.-Butyl sind;
- R'₁₅: Wasserstoff ist;
- X': eine direkte Bindung;
- Y': -O-; und
- Z': eine direkte Bindung, -CH₂- oder -CH(CH₃)- ist.

Im Speziellen sind zur Mischung mit Verbindungen der Formel (1) die konkreten Phosphorverbindungen der Formeln C'1 bis C'12 zu nennen: 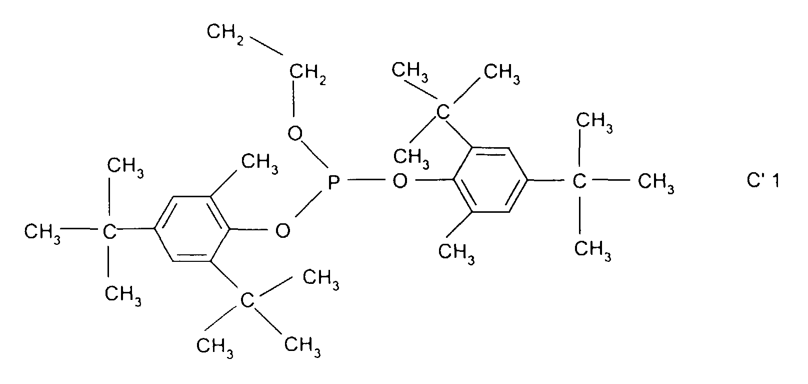 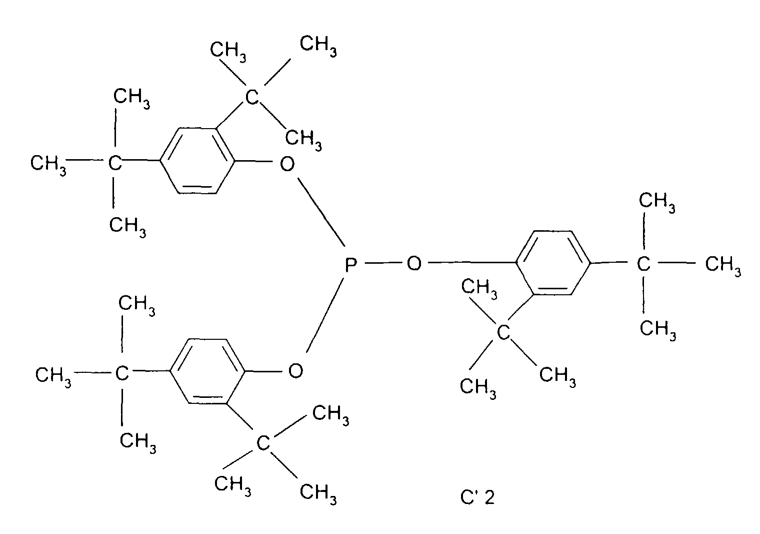 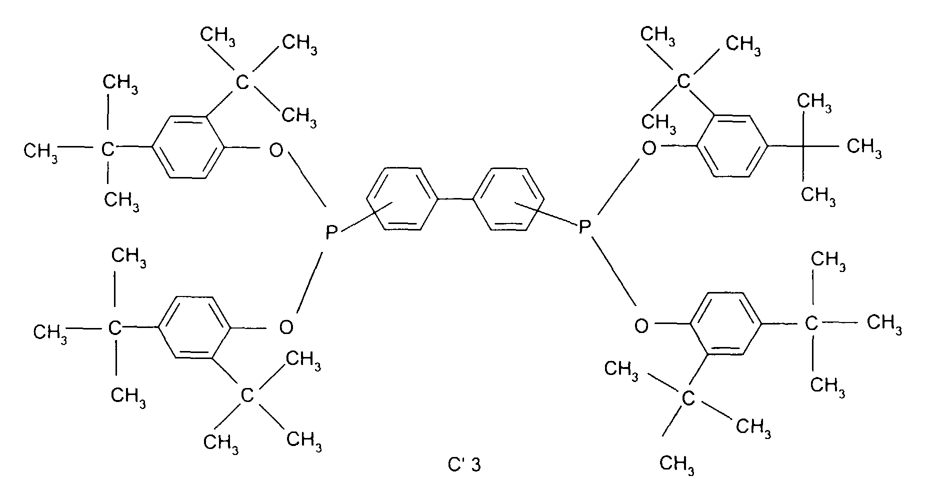 In der Formel C'3 befinden sich die beiden Phosphor-Substituenten hauptsächlich in 4- bzw. 4'-Stellung des Biphenylgrundgerüsts

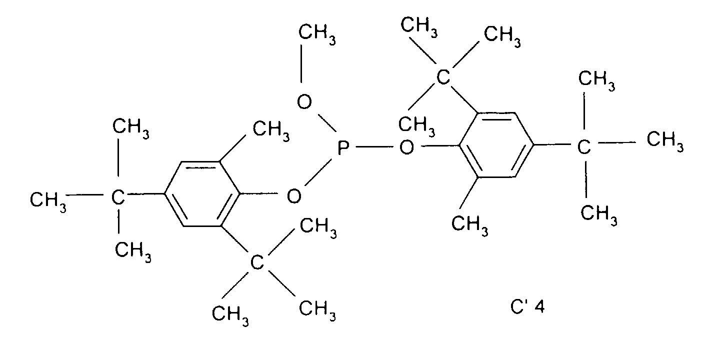

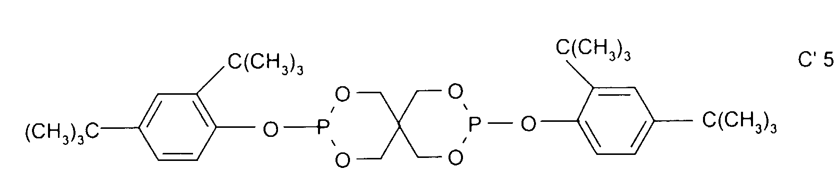

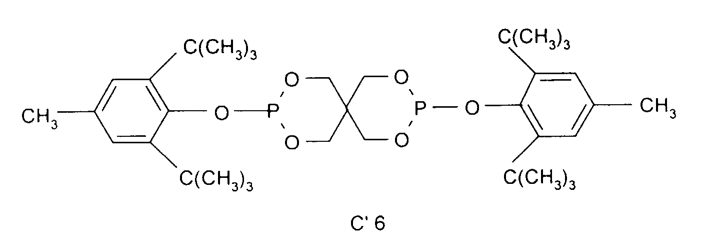

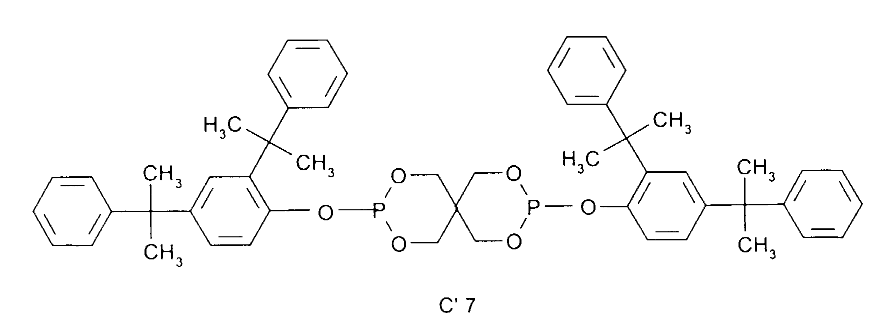

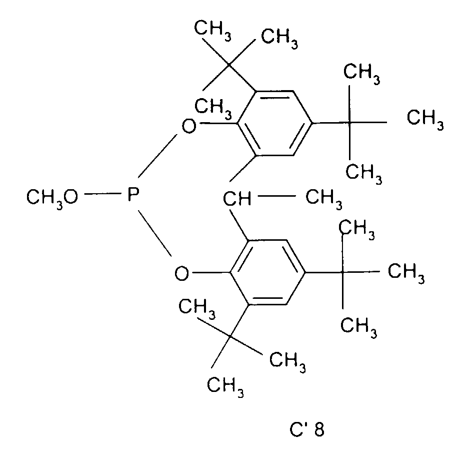

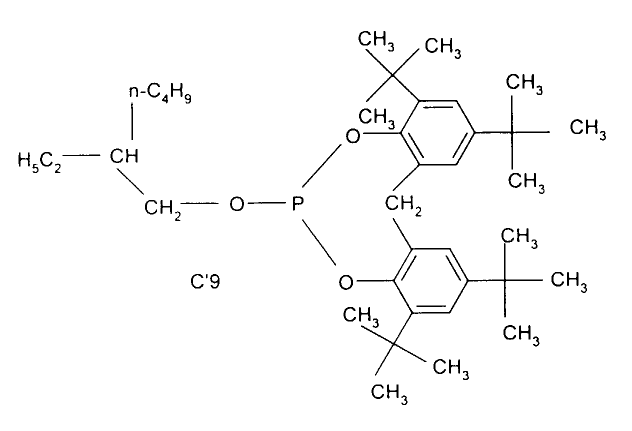

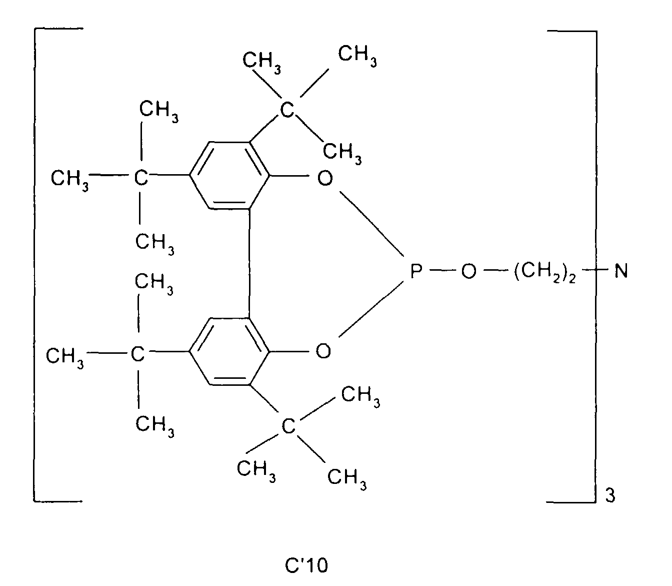

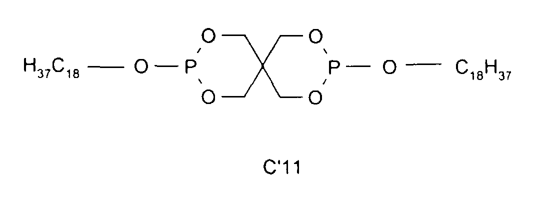

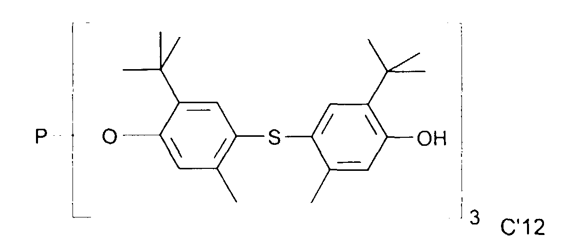

Die genannten Phosphite und Phosphonite sind bekannte Verbindungen, sie sind zum Teil kommerziell erhältlich.

Bei folgenden Stabilisatormischungen handelt es sich um besonders geeignete Ausführungsformen der Erfindung:
Verbindung (1) und ®Irgafos 38,
Verbindung (1) und ®Irgafos 12,
Verbindung (1) und ®Hostanox PAR 24,
Verbindung (1) und ®Hostanox OSP 1,
Verbindung (1) und ®Irgafos P-EPQ,
Verbindung (1) und ®Ultranox 626,
Verbindung (1) und ®Ultranox 618,
Verbindung (1) und ®Mark PEP-36 (von Asahi Denka),
Verbindung (1) und ®Mark HP10 (von Asahi Denka),
Verbindung (1) und ®Doverphos 9228

Die Kombination von Verbindungen (1) mit Phosphiten und Phosphoniten eignet sich auch in hervorragender Weise in dem Sinne, dass das Phosphit bzw. Phosphonit die Wirkung der Verbindungen (1) bei der Stabilisierung von organischem Material in einer synergistischen Art und Weise unterstützt. Synergistische Effekte dieser Art sind in EP-A-359 276 und EP-A-567 117 beschrieben. Besonders geeignet sind Mischungen der Verbindungen (1) mit Phosphiten bzw. Phosphoniten der Formeln C'1 bis C'12.

Die Verbindungen (1) eignen sich auch in hervorragender Weise, mit Phosphit bzw. Phosphonit, und/oder einem sterisch gehinderten Phenol und/oder einem Säurefänger kombiniert zu werden. Besonders geeignet ist die Kombination der Verbindungen (1) in Mischungen mit Phosphit bzw. Phosphonit, Phenol und Säurefänger, in einer Art und Weise, wie sie DE-A-19 537 140 beschreibt.

Die Verbindungen (1) sowie die vorstehend beschriebenen Gemische eignen sich auch mit anderen Stabilisatoren, insbesondere mit Lichtschutzmitteln, wie z. B. aus der Klasse der UV-Absorber (2-Hydroxybenzophenone oder 2-Hydroxyphenyl-benztriazole, Zimtsäurederivate, Oxanilide und Triazin-Derivate) und/oder Nickel-Quencher, in einer synergistischen Art und Weise kombiniert zu werden.

Bei den vorstehend beschriebenen Gemischen kann der Anteil an Verbindungen der Formel (1) zwischen 1 und 99 Gew.-% liegen.

Die Verbindungen (1) eignen sich auch zum Einsatz in Kombination mit Zeolithen oder Hydrotalciten, wie z.B. ®DHT4A analog EP-A-429 731.

Die Verbindungen (1) sowie die vorstehend beschriebenen Mischungen können auch mit einem oder mehreren N,N-dialkylsubstituierten Hydroxylaminen kombiniert werden, vorzugsweise mit N,N-Dioctadecylhydroxylamin.

Weiterhin können die Verbindungen (1) mit einem oder mehreren basischen oder sonstigen säurebindenden Costabilisatoren aus der Gruppe der Metallcarboxylate, -oxide, -hydroxide, -carbonate, und/oder Zeolithe, und/oder Hydrotalcite, kombiniert werden.

Bevorzugte Costabilisatoren sind Calciumstearat, und/oder Magnesiumstearat, und/oder Magnesiumoxid, und/oder Zinkoxid und/oder carbonathaltiges Zinkoxid und/oder Hydrotalcite sowie Metallsalze anderer organischer Säuren wie z.B. Calciumlactat.

Besonders bevorzugte Costabilisatoren sind ®Zinkoxid aktiv, ®Zinkoxid transparent und/oder eines der Hydrotalcite ®DHT 4A, ®DHT4 A2, ®Kyowaad 200, ®Kyowaad 300, ®Kyowaad 400, ®Kyowaad 500, ®Kyowaad 600, ®Kyowaad 700, ®Kyowaad 1000, ®Kyowaad 2000.

Erfindungsgemäss werden die Verbindungen der Formel (1), gegebenenfalls in Kombinationen mit anderen Stabilisatoren, zur Stabilisierung von organischen Materialien, insbesonders polymeren organischen Materialien, ganz besonders von synthetischen polymeren organischen Materialien (Kunststoffen) eingesetzt. Verbindungen der Formel (1), gegebenenfalls in Kombinationen mit anderen Stabilisatoren, werden erfindungsgemäss während deren Herstellung und Verarbeitung bzw. während deren Langzeitgebrauch verwendet, insbesonders, wenn das stabilisierte Material dem Einfluss von Strahlung, Licht oder Wärme in besonderem Masse ausgesetzt ist.

### Beispiele für derartige Kunststoffe sind:

1. Polymere von Mono- und Diolefinen, beispielsweise Polypropylen, Polyisobutylen, Polybutylen, Polybuten-1, Poly-4-methylpenten-1, Polyisopropren oder Polybutadien sowie Polymerisate von Cycloolefinen wie z.B. von Cyclopenten oder Norbornen; ferner Polyethylen (das gegebenenfalls vernetzt sein kann); z.B. Polyethylen hoher Dichte (HDPE), Polyethylen hoher Dichte und hoher Molmasse (HDPE-HMW), Polyethylen hoher Dichte und ultrahoher Molmasse (HDPE-UHMW), Polyethylen mittlerer Dichte (MDPE), Polyethylen niederer Dichte (LDPE), lineares Polyethylen niederer Dichte (LLPE), verzweigtes Polyethylen niederer Dichte (VLDPE).
   Polyolefine, d.h. Polymere von Monoolefinen, wie sie beispielshaft im vorstehenden Absatz erwähnt sind, insbesondere Polyethylen und Polypropylen, können nach verschiedenen Verfahren hergestellt werden, insbesondere nach den folgenden Methoden:
   a) radikalisch (gewöhnlich bei hohem Druck und hoher Temperatur)
   b) mittels Katalysator, wobei der Katalysator gewöhnlich ein oder mehrere Metalle der Gruppe IVb, Vb, VIb oder VIII enthält. Diese Metalle besitzen gewöhnlich einen oder mehrere Liganden wie Oxide, Halogenide, Alkoholate, Ester, Ether, Amine, Alkyle, Alkenyle und/oder Aryle, die entweder π- oder σ-koordiniert sein können. Diese Metallkomplexe können frei oder auf Träger fixiert sein, wie beispielsweise auf aktiviertem Magnesiumchlorid, Titan(III)chlorid, Aluminiumoxid oder Siliziumoxid. Diese Katalysatoren können im Polymerisationsmedium löslich oder unlöslich sein. Die Katalysatoren können als solche in der Polymerisation aktiv sein, oder es können weitere Aktivatoren verwendet werden, wie beispielsweise Metallalkye, Metallhydride, Metallalkylhalogenide, Metallalkyloxide oder Metallalkyloxane, wobei die Metalle Elemente der Gruppen la, lla und/oder IIIa sind. Die Aktivatoren können beispielsweise mit weiteren Ester-, Ether-, Amin- oder Silylether-Gruppen modifiziert sein. Diese Katalysatorsysteme werden gewöhnlich als Philipps, Standard Oil Indiana, Ziegler (-Natta), TNZ (DuPont), Metallocen oder Single Site Katalysatoren (SSC) bezeichnet.
2. Mischungen der unter 1) genannten Polymeren, z. B. Mischungen von Polypropylen mit Polyisobutylen, Polyethylen mit Polyisobutylen, Polypropylen mit Polyethylen (z.B. PP/HDPE/LDPE) und Mischungen verschiedener Polyethylentypen (z.B. LDPE/HDPE) untereinander.
3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-lsobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-lsopren-Copolymere, Ethylen-Alkylacrylat-Copolymere, Ethylen-Alkylmethacrylat-Copolymere, Ethylen-Vinylacetat-Copolymere und deren Copolymere mit Kohlenstoffmonoxid, oder Ethylen-Acrylsäure-Copolymere und deren Salze (lonomere), sowie Terpolymere von Ethylen mit Propylen und einem Dien, wie Hexadien, Dicyclopentadien oder Ethylidenorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere, LLDPE/Ethylen-Acrylsäure-Copolymere und alternierend oder statistisch aufgebaute Polyalkylen/Kohlenstoffmonoxid-Copolymere und deren Mischungen mit anderen Polymeren wie z.B. Polyamiden.
4. Kohlenwasserstoffharze (z.B. C₅-C₉) inklusive hydrierte Modifikationen davon (z.B. Klebrigmacherharze) und Mischungen von Polyalkylenen und Stärke.
5. Polystyrol, Poly(p-methylstyrol), Poly-(α-methylstyrol).
6. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Butadien-Alkylacrylat und -methacrylat, Styrol-Maleinsäureanhydrid, Styrol-Acrylnitril-Methacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-lsopren-Styrol, Styrol-Ethylen/Butylen-Styrol oder Styrol-Ethylen/Propylen-Styrol.
7. Pfropfcopolymere von Styrol oder α-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien; Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 6) genannten Copolymeren, wie sie z.B. als sogenannte ABS-, MBS-, ASA- oder AES-Polymere bekannt sind.
8. Halogenhaltige Polymere, wie z.B. Polychloropren, Chlorkautschuk, chloriertes und bromiertes Copolymer aus Isobutylen-Isopren (Halobutylkautschuk), chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.
9. Polymere, die sich von α,ss-ungesättigten Säuren und deren Derivate ableiten, wie Polyacrylate und Polymethacrylate, mit Butylacrylate schlagzäh modifizierte Polymethylmethacrylate, Polyacrylamide und Polyacrylnitrile.
10. Copolymere der unter 9) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.
11. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.
12. Homo- und Copolymere von cyclischen Ethern, wie Polyalkylenglykole, Polyethylenoxyd, Polypropylenoxyd oder deren Copolymere mit Bisglycidethern.
13. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.
14. Polyphenylenoxide und -sulfide und deren Mischungen mit Styrolpolymeren oder Polyamiden.
15. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.
16. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, 12/12, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylen-terephthalamid oder Poly-m-phenylen-isopthalamid. Block-Copolymere der vorstehend genannten Polyamide mit Polyolefinen, Olefin-Copolymeren, lonomeren, oder chemisch gebundenen oder gepropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").
17. Polyharnstoffe, Polyimide, Polyamidimide, Polyetherimide, Polyesteramide, Polyhydantoine und Polybenzimidazole.
18. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.
19. Polycarbonate und Polyestercarbonate.
20. Polysulfone, Polyethersulfone und Polyetherketone.
21. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.
22. Trocknende und nicht-trocknende Alkydharze.
23. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.
24. Vernetzbare Acrylharze, die sich von substituierten Acrylsäurestern ableiten, wie z.B. von Epoxyacrylaten, Urethan-acrylaten oder Polyester-acrylaten.
25. Alkydharze, Polyesterharze und Acrylharze, die mit Melaminharzen, Harnstoffharzen, Isocyanaten, Isocyanuraten, Polyisocyanaten oder Epoxidharzen vernetzt sind.
26. Vernetzte Epoxidharze, die sich von aliphatischen, cycloaliphatischen, heterocyclischen oder aromatischen Glycidylverbindungen ableiten, z.B. Produkte von Bisphenol-A-diglycidylethern, Bisphenol-F-diglycidylethern, die mittels üblichen Härtern wie z.B. Anhydriden oder Aminen mit oder ohne Beschleunigern vernetzt werden.
27. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.
28. Mischungen (Polyblends) der vorgenannten Polymeren, wie z.B. PP/EPDM, Polyamid/EPDM oder ABS, PVC/EVA, PVC/ABS, PVC/MBS, PC/ABS, PBT/ABS, PC/ASA, PC/PBT, PVC/CPE, PVC/Acrylate, POM/thermoplastisches PUR, PC/thermoplastisches PUR, POM/Acrylat, POM/MBS, PPO/MBS, PPO/HIPS, PPO/PA 6.6 und Copolymere, PA/HDPE, PA/PP, PA/PPO, PBT/PC/ABS oder PBT/PET/PC.
29. Natürliche und synthetische organische Stoffe, die reine monomere Verbindungen oder Mischungen von solchen darstellen, beispielsweise Mineralöle, tierische oder pflanzliche Fette, Öle und Wachse, oder Öle, Wachse und Fette auf Basis synthetischer Ester (z.B. Phthalate, Adipate, Phosphate oder Trimellitate), sowie Abmischungen synthetischer Ester mit Mineralölen in beliebigen Gewichtsverhältnissen, wie sie z.B. als Spinnpräparationen Anwendung finden, sowie deren wässrige Emulsionen.
30. Wässrige Emulsionen natürlicher oder synthetischer Kautschuke, wie z.B. Naturkautschuk-Latex oder Latices von carboxylierten Styrol-Butadien-Copolymeren.

Das erfindungsgemäss durch die Verbindungen der Formel (1) oder durch eine geeignete Kombination mit diesen Verbindungen stabilisierte organische Material kann gegebenenfalls noch weitere Additive enthalten, beispielsweise Antioxidantien, Lichtstabilisierungsmittel, Metalldesaktivatoren, antistatische Mittel, flammhemmende Mittel, Gleitmittel, Nukleierungsmittel, Säurefänger (basische Costabilisatoren), Pigmente und Füllstoffe. Antioxidantien und Lichtstabilisatoren, die neben den Verbindungen (1) oder Kombinationen zugesetzt werden, sind beispielsweise Verbindungen auf der Basis sterisch gehinderter Amine oder sterisch gehinderter Phenole oder Schwefel oder Phosphor enthaltende Costabilisatoren. Als geeignete, zusätzlich in Kombination einsetzbare Additive sind beispielsweise Verbindungen verwendbar, wie sie nachfolgend aufgeführt sind:

### 1. Antioxidantien

1.1 Alkylierte Monophenole, z.B. 2,6 Di-tert-butyl-4-methylphenol, 2-Butyl-4,6-dimethylphenol, 2,6-Di-tert-butyl-4-ethylphenol, 2,6-Di-tert-butyl-4-n-butylphenol, 2,6-Di-tert-butyl-4-isobutylphenol, 2,6-Di-cyclopentyl-4-methylphenol, 2-(a-Methyl-cyclohexyl)-4,6-dimethyl-phenol, 2,6-Di-octadecyl-4-methylphenol, 2,4,6-Tri-cyclohexylphenol, 2,6-Di-tert-butyl-4-methoxymethylphenol, lineare oder in der Seitenkette verzweigte Nonylphenole, wie z. B. 2,6-Dinonyl-4-methylphenol, 2,4-Dimethyl-6(1'-methyl-undec-1'-yl)-phenol, 2,4-Dimethyl-6-(1'-methyl-heptadec-1'-yl)-phenol, 2,4-Dimethyl-6-(1'-methyl-tridec-1'-yl)-phenol und Mischungen davon.
1.2 Alkylthiomethylphenole, z.B. 2,4-Di-octylthiomethyl-6-tert-butylphenol, 2,4-Dioctyl-thiomethyl-6-methylphenol, 2,4-Di-octylthiomethyl-6-ethylphenol, 2,6-Di-dodecylthiomethyl-4-nonyl-phenol.
1.3 Hydrochinone und alkylierte Hydrochinone, z.B. 2,6-Di-tert-butyl-4-methoxyphenol, 2,5-Di-tert-butyl-hydrochinon, 2,5-Di-tert-amyl-hydrochinon, 2,6-Diphenyl-4-octadecyloxyphenol, 2,6-Di-tert-butyl-hydrochinon, 2,5-Di-tert-butyl-4-hydroxyanisol, 3,5-Di-tert-butyl-4-hydroxyanisol, 3,5-Di-tert-butyl-4-hydroxyphenyl-stearat, Bis-(3,5-di-tert-butyl-4-hydroxy-phenyl)adipat.
1.4 Hydroxylierte Thiodiphenylether, z.B. 2,2'-Thio-bis-(6-tert-butyl-4-methylphenol), 2,2'-Thio-bis-(4-octylphenol), 4,4'-Thio-bis-(6-tert-butyl-3-methylphenol), 4,4'-Thio-bis-(6-tert-butyl-2-methyl-phenol), 4,4'-Thio-bis-(3,6-di-sec.-amylphenol), 4,4'-Bis-(2,6-dimethyl-4-hydroxyphenyl)-disulfid.
1.5 Alkyliden-Bisphenole, z.B. 2,2'-Methylen-bis-(6-tert-butyl-4-methylphenol), 2,2'-Methylen-bis-(6-tert-butyl-4-ethylphenol), 2,2'-Methylen-bis-[4-methyl-6-(α-methylcyclo-hexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert-butylphenol), 2,2'-Ethyliden-bis-(4,6-di-tert-butylphenol), 2,2'-Ethyliden-bis-(6-tert-butyl-4-isobutylphenol), 2,2'-Methylen-bis-[6-(α-methylbenzyl)-4-nonyl-phenol], 2,2'-Methylen-bis-[6-(α,α-dimethylbenzyl)-4-nonylphenol], 4,4'-Methylen-bis-(2,6-di-tert-butylphenol), 4,4'-Methylen-bis-(6-tert-butyl-2-methylphenol), 1,1-Bis-(5-tert-butyl-4-hydroxy-2-methylphenyl)-butan, 2,6-Bis-(3-tert-butyl-5-methyl-2-hydroxybenzyl)-4-methyl-phenol, 1,1,3-Tris-(5-tert-butyl-4-hydroxy-2-methyl-phenyl)-butan, 1,1-Bis-(5-tert-butyl-4-hydroxy-2-methylphenyl)-3-n-dodecylmercaptobutan, Bis-(3-tert-butyl-4-hydroxy-5-methylphenyl)-dicyclopentadien, Bis-[2-(3'-tert-butyl-2'-hydroxy-5'-methyl-benzyl)-6-tert-butyl-4-methyl-phenyl]-terephthalat, 1,1-Bis-(3,5-dimethyl-2-hydroxyphenyl)-butan, 2,2-Bis-(3,5-di-tert-butyl-4-hydroxyphenyl)-propan, 2,2-Bis-(5-tert-butyl-4-hydroxy-2-methylphenyl)-4-n-dodecylmercaptobutan, 1,1,5,5-Tetra-(5-tert-butyl-4-hydroxy-2-methyl-phenyl)-pentan, Ethylenglycol-bis[3,3-bis(3'-tert-butyl-4'-hydroxyphenyl)butyrat].
1.6 O-, N- und S-Benzylverbindungen, z.B. 3,5,3',5'-Tetra-tert-butyl-4,4'-dihydroxydibenzylether, Octadecyl-4-hydroxy-3,5-dimethylbenzyl-mercaptoacetat, Tris-(3,5-di-tert-butyl-4-hydroxybenzyl)-amin, Bis-(4-tert-butyl-3-hydroxy-2,6-dimethylbenzyl)-dithioterephthalat, Bis-(3,5-di-tert-butyl-4-hydroxybenzyl)-sulfid, lsooctyl-3,5-di-tert-butyl-4-hydro-xybenzyl-mercaptoacetat, Tridecyl-4-hydroxy-3,5-di-tert-butylbenzylmercaptoacetat.
1.7 Hydroxybenzylierte Malonate, z.B. Dioctadecyl-2,2-bis-(3,5-di-tert-butyl-2-hydroxybenzyl)-malonat, Di-octadecyl-2-(3-tert-butyl-4-hydroxy-5-methylbenzyl)-malonat, Didodecylmercaptoethyl-2,2-bis-(3,5-di-tert-butyl-4-hydroxybenzyl)-malonat, Di-[4(1,1,3,3-tetramethylbutyl)-phenyl]-2,2-bis(3,5-di-tert-butyl-4-hydroxybenzyl)malonat.
1.8 Hydroxybenzyl-Aromaten, z. B. 1,3,5-Tris-(3,5-di-tert-butyl-4-hydroxybenzyl)-2,4,6-trimethylbenzol, 1,4-Bis-(3,5-di-tert-butyl-4-hydroxybenzyl)-2,3,5,6-tetramethylbenzol, 2,4,6-Tris-(3,5-di-tert-butyl-4-hydroxybenzyl)-phenol.
1.9 Triazinverbindungen, z.B. 2,4-Bis-octylmercapto-6-(3,5-di-tert-butyl-4-hydroxyanilino)-1,3,5-triazin, 2-Octylmercapto-4,6-bis-(3,5-di-tert-butyl-4-hydroxyanilino)-1,3,5-triazin, 2-Octylmercapto-4,6-bis-(3,5-di-tert-butyl-4-hydroxyphenoxy)-1,3,5-triazin, 2,4,6-Tris-(3,5-di-tert-butyl-4-hydroxyphenoxy)-1,2,3-triazin, 1,3,5-Tris-(3,5-tert-butyl-4-hydroxybenzyl)-isocyanurat, 1,3,5-Tris-(4-tert-butyl-3-hydroxy-2,6-dimethylbenzyl)-isocyanurat, 2,4,6-Tris-(3,5-di-tert-butyl-4-hydroxyphenylethyl)-1,3,5-triazin, 1,3,5-Tris-(3,5-di-tert-butyl-4-hydroxyphenylpropionyl)-hexahydro-1,3,5-triazin, 1,3,5-Tris-(3,5-dicyclohexyl-4-hydroxybenzyl)-isocyanurat.
1. 10 Benzylphosphonate, z.B. Dimethyl-2,5-di-tert-butyl-4-hydroxybenzyl-phosphonat, Diethyl-3,5-di-tert-butyl-4-hydroxybenzylphosphonat, Dioctadecyl-3,5-di-tert-butyl-4-hydroxybenzylphosphonat, Dioctadecyl-5-tert-butyl-4-hydroxy-3-methylbenzylphosphonat, Ca-Salz des 3,5-di-tert-butyl-4-hydroxybenzyl-phosphonsäure-monoethylesters.
1.11 Acylaminophenole, z.B. 4-Hydroxy-laurinsäureanilid, 4-Hydroxy-stearinsäureanilid, N-(3,5-di-tert-butyl-4-hydroxyphenyl)-carbaminsäureoctylester.
1.12 Ester der ss-(3,5-Di-tert-butyl-4-hydroxyphenyl)-propionsäure mit ein- oder mehrwertigen Alkoholen, wie mit Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2,2,2]-octan.
1.13 Ester der ss-(5-tert-Butyl-4-hydroxy-3-methylphenyl)-propionsäure mit ein- oder mehrwertigen Alkoholen, wie z.B. mit Methanol, Ethanol, n-Octanol, i-Octanol, Octadecanol, 1,6-Hexandiol, 1,9-Nonandiol, Ethylenglycol, 1,2-Propandiol, Neopentylglycol, Thiodietyhlenglycol, Diethylenglycol, Triethylenglycol, Pentaerythrit, Tris-(hydroxyethyl)-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2,2,2]-octan.
1.14 Ester der ss-(3,5-Dicyclohexyl-4-hydroxyphenyl)-propionsäure mit ein- oder mehrwertigen Alkoholen, wie z.B. Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-(2,2,2)-octan.
1.15 Ester der 3,5-Di-tert-butyl-4-hydroxyphenylessigsäure mit ein- oder mehrwertigen Alkoholen, wie z.B. mit Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2,2,2]-octan.
1.16 Ester der 3,3-Bis-(3'-tert-butyl-4'-hydroxyphenyl)-buttersäure mit ein- oder mehrwertigen Alkoholen, wie z.B. mit Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2,2,2]-octan.
1.17 Amide der ss-(3,5-Di-tert-butyl-4-hydroxyphenyl)-propionsäure, wie z.B. N,N'-Bis-(3,5-di-tert-butyl-4-hydroxyphenylpropionyl)-hexamethylendiamin, N,N' -Bis-(3,5-di-tert-butyl-4-hydroxyphenylpropionyl)-trimethylendiamin, N,N'-Bis-(3,5-di-tert-butyl-4-hydroxy-phenylpropionyl)-hydrazin.
1.18. Chroman-Derivate wie z.B. Tocopherol (α-Tocopherol, ss-Tocopherol, γ-Tocopherol, δ-Tocopherol) sowie Mischungen davon (Vitamin E) und weitere Mischungen untereinander .
1.19. Ascorbinsäure (Vitamin C).
1.20. Aminische Antioxidantien, wie z. B. N, N'-Di-isopropyl-p-phenylendiamin, N,N'-Di-sec-butyl-p-phenylendiamin, N,N'-Bis(1,4-dimethyl-pentyl)-p-phenylendiamin, N,N'-Bis(1-ethyl-3-methyl-pentyl)-p-phenylendiamin, N,N'-Bis(1-methyl-heptyl)-p-phenylendiamin, N,N'-Dicyclohexyl-p-phenylendiamin, N,N'-Diphenyl-p-phenylendiamin, N,N'-Di(naphthyl-2-)-p-phenylendiamin, N-Isopropyl,N'-phenyl-p-phenylendiamin, N-(1,3-Dimethylbutyl)-N'-phenyl-p-phenylendiamin, N-(1-Methyl-heptyl)-N'-phenyl-p-phenylendiamin, N-Cyclohexyl-N'-phenyl-p-phenylendiamin, 4-(p-Toluolsulfonamido)-diphenylamin, N,N'-Dimethyl-N,N'-di-sec-butyl-p-phenylendiamin, Diphenylamin, N-Allyldiphenylamin, 4-lsopropoxy-diphenyl-amin, N-Phenyl-1-naphthylamin, N-(4-tert-octylphenyl)-1-naphthylamin, N-Phenyl-2-naphthylamin, octyliertes Diphenylamin, z. B. p,p'-Di-tert-octyl-diphenylamin, 4-n-Butylaminophenol, 4-Butyrylaminophenol, 4-Nonanoylaminophenol, 4-Dodecanoylaminophenol, 4-Octadecanoylaminophenol, Di(4-methoxyphenyl)-amin, 2,6-Di-tert-butyl-4-dimethylamino-methyl-phenol, 2,4'-Diaminodiphenylmethan, 4,4'-Di-amino-diphenylmethan, N,N,N',N'-Tetramethyl-4,4'diaminodiphenylmethan, 1,2-Di-[(2-methyl-phenyl)-amino]-ethan, 1,2-Di-(phenylamino)-propan, (o-Tolyl)-biguanid, Di-[4-(1',3'-di-methyl-butyl)-phenyl]amin, tert-octyliertes N-Phenyl-1-naphthylamin, Gemisch aus mono- und dialkylierten tert Butyl-/tert-Octyldiphenylaminen, Gemisch aus mono- und dialkylierten Nonyldiphenylaminen, Gemisch aus mono- und dialkylierten Do-decyldiphenylaminen, Gemisch aus mono- und dialkylierten Isopropyl/Isohexyl-di-phenylaminen, Gemisch aus mono- und dialkylierten tert-Butyldiphenylaminen, 2,3-Di-hydro-3,3-dimethyl-4H-1,4-benzothiazin, Phenothiazin, Gemisch aus mono- und di-alkylierten tert-Butyl/tert-Octylphenothiazinen, Gemisch aus mono- und dialkylierten tert-Octyl-phenothiazinen, N-Allylphenothiazin, N,N,N',N'-Tetra-phenyl-1,4-diaminobut-2-en, N,N-Bis(2,2,6,6-tetramethylpiperidin-4-yl)-hexamethylendiamin, Bis-(2,2,6,6-tetra-methylpiperidin-4-yl)-sebacat, 2,2,6,6-Tetramethylpiperidin-4-on, 2,2,6,6-Tetramethyl-piperidin-4-ol.

### 2. UV-Absorber und Lichtschutzmittel

2.1 2-(2'-Hydroxyphenyl)-benztriazole, wie z.B. 2-(2'-Hydroxy-5'-methylphenyl)-benztriazol, 2-(3',5'-Di-tert-butyl-2'-hydroxyphenyl)-benztriazol, 2-(5'-tert-Butyl-2'-hydroxyphenyl)-benztriazol, 2-[2'-Hydroxy-5'-(1,1,3,3-tetramethylbutyl)phenyl]-benztriazol, 2-(3',5'-Di-tert-butyl-2'-hydroxy-phenyl)-5-chlor-benztriazol,2-(3' -tert-Butyl-2'-hydroxy-5'-methylphenyl)-5-chlor-benztriazol, 2-(3'-sec-Butyl-5'-tert-butyl-2'-hydroxyphenyl)-benztriazol, 2-(2'-Hydroxy-4'-octoxyphenyl)-benztriazol, 2-(3',5'-Di-tert-amyl-2'-hydroxyphenyl)-benztriazol, 2-(3',5'-Bis-(α,α-dimethylbenzyl)-2'-hydroxyphenyl)-benztriazol, Mischung aus 2-(3'-tert-Butyl-2'-hydroxy-5'-(2-octyloxycarbonylethyl)-phenyl)-5-chlor-benztriazol, 2-(3'-tert-Butyl-5'-[2-(2-ethylhexyloxy)-carbonylethyl]-2'-hydroxyphenyl)-5-chlor-benztriazol, 2-(3'-tert-Butyl-2'-hydroxy-5'-(2-methoxycarbonylethyl)phenyl)-5-chlor-benztriazol, 2-(3'-tert-Butyl-2'-hydroxy-5'-(2-methoxycarbonylethyl)phenyl)-benztriazol, 2-(3'-tert-Butyl-2'-hydroxy-5'-(2-octyloxycarbonylethyl)phenyl)-benztriazol, 2-(3'-tert-Butyl-5'-[2-(2-ethylhexyloxy)carbonylethyl]-2'-hydroxyphenyl)-benztriazol, 2-(3'-Dodecyl-2'-hydroxy-5'-methylphenyl)-benztriazol, und 2-(3'-tert-Butyl-2'-hydroxy-5'-(2-isooctyloxycarbonylethyl)phenyl-benztriazol, 2,2'-Methylen-bis-[4-(1,1,3,3-tetramethylbutyl)-6-benztriazol-2-yl-phenol]; Umesterungsprodukt von 2-[3'-tert-Butyl-5'(2-methoxycarbonylethyl)-2'-hydroxyphenyl]-benztriazol mit Polyethylenglycol 300; [R-CH₂CH₂-COO(CH₂)₃]-₂ mit R = 3'-tert-Butyl-4'-hydroxy-5'-2H-benztriazol-2-yl-phenyl.
2.2 2-Hydroxybenzophenone, wie z.B. das 4-Hydroxy-, 4-Methoxy-, 4-Octoxy, 4-Decyloxy, 4-Dodecyloxy-, 4-Benzyloxy-, 4,2',4'-Trihydroxy-, 2'-Hydroxy-4,4'-dimethoxy-Derivat.
2.3 Ester von gegebenenfalls substituierten Benzoesäuren, wie z.B. 4-tert-Butyl-phenylsalicylat, Phenylsalicylat, Octylphenylsalicylat, Dibenzoylresorcin, Bis-(4-tert-butylbenzoyl)-resorcin, Benzoylresorcin, 3,5-Di-tert-butyl-4-hydroxybenzoesäure-2,4-di-tert-butylphenylester, 3,5-Di-tert-butyl-4-hydroxybenzosäurehexadecylester, 3,5-Di-tert-butyl-4-hydroxy-benzoesäureoctadecylester, 3,5-Di-tert-butyl-4-hydroxybenzoesäure-2-methyl-4,6-di-tert-butylphenylester.
2.4 Acrylate wie z.B. α-Cyan-ss,ss-diphenylacrylsäureethylester bzw. -isooctylester, α-Carbomethoxyzimtsäuremethylester, α-Cyano-ss-methyl-p-methoxyzimtsäuremethylester bzw. -butylester, α-Carbomethoxy-p-methoxy-zimtsäuremethylester, N-(ss-Carbo-methoxy-ss-cyanovinyl)-2-methylindolin.
2.5 Nickelverbindungen wie z.B. Nickelkomplexe des 2,2'-Thio-bis-[4-(1,1,3,3 tetramethylbutyl)-phenols] wie der 1:1 oder der 1:2 Komplex, gegebenenfalls mit zusätzlichen Liganden, wie n-Butylamin, Triethanolamin oder N-Cyclohexyldiethanolamin, Nickeldibutyldithiocarbamat, Nickelsalze von 4-Hydroxy-3,5-di-tert-butyl-benzylphosphonsäuremonoalkylestern, wie vom Methyl- oder Ethylester, Nickelkomplexe von Ketoximen, wie von 2-Hydroxy-4-methyl-phenylundecylketoxim, Nickelkomplexe des 1-Phenyl-4-lauroyl-5-hydroxy-pyrazols, gegebenenfalls mit zusätzlichen Liganden.
2.6 Sterisch gehinderte Amine, wie z.B. Bis-(2,2,6,6-tetramethyl-piperidin-4-yl)-sebacat, Bis-(2,2,6,6-tetramethylpiperidin-4-yl)-glutarat, Bis-(2,2,6,6-tetramethyl-piperidin-4-yl)-succinat, Bis-(1,2,2,6,6-pentamethylpiperidin-4-yl)-sebacat, Bis-(1,2,2,6,6-pentamethylpiperidin-4-yl)-glutarat, n-Butyl-3,5-di-tert-butyl-4-hydroxybenzylmalonsäure-bis-(1,2,2,6,6-pentamethylpiperidyl)-ester, 2,2,6,6-Tetramethylpiperidylbehenat, 1,2,2,6,6-Pentamethylpiperidylbehenat, Kondensations-produkt aus 1-Hydroxyethyl-2,2,6,6-tetra-methyl-4-hydroxypiperidin und Bernsteinsäure, Kondensationsprodukt aus N,N'-Bis-(2,2,6,6-Tetramethyl-4-piperidyl)-hexamethylendiamin und 4-tert-Octylamino-2,6-di-chlor-1,3,5-s-triazin, Tris-(2,2,6,6-tetramethyl-4-piperidyl)-nitrilotriacetat, Tetrakis-(2,2,6,6-tetramethyl-4-piperidyl)-1,2,3,4-butantetraoat, 1,1'-(1,2-Ethandiyl)-bis-(3,3,5,5-tetramethylpiperazinon), 4-Benzoyl-2,2,6,6-tetramethylpiperidin, 4-Stearyloxy-2,2,6,6-tetramethylpiperidin, 4-Stearoyloxy-2,2,6,6-tetramethylpiperidin, 4-Stearyloxy-1,2,2,6,6-pentamethylpiperidin, 4-Stearoyloxy-1,2,2,6,6-pentamethylpiperidin, Bis-(1,2,2,6,6-pentamethylpiperidyl)-2-n-butyl-2-(2-hydroxy-3,5-di-tert-butylbenzyl)-malonat, Bis-(1,2,2,6,6-pentamethylpiperidyl)-2-n-butyl-2-(4-hydroxy-3,5-di-tert-butylbenzyl)-malonat, 3-n-Octyl-7,7,9,9-tetramethyl-1,3,8-triazaspiro [4,5] decan-2,4-dion, Bis-(1-octyloxy-2,2,6,6-tetramethylpiperidyl)-sebacat, Bis-(1-octyloxy-2,2,6,6-tetramethylpiperidyl)-succinat, Kondensationsprodukt aus N,N'-Bis-(2,2,6,6-tetramethyl-4-piperidyl)-hexamethylendiamin und 4-Morpholino-2,6-dichlor-1,3,5-triazin, Kondensationsprodukt aus N, N'-Bis(2,2,6,6-tetramethyl-4-piperidyl)-hexamethylendiamin und 4-Cyclohexylamino-2,6-dichlor-1,3,5-triazin, Kondensationsprodukt aus 2-Chlor-4,6-di-(4-n-butylamino-2,2,6,6-tetramethyl-piperidyl)-1,3,5-triazin und 1,2-Bis-(3-aminopropylamino)ethan, Kondensationsprodukt aus 2-Chlor-4,6-di-(4-methoxypropylamino-2,2,6,6-tetramethylpiperidyl)-1,3,5-triazin und 1,2-Bis-(3-aminopropylamino)ethan, Kondensationsprodukt aus 2-Chlor-4,6-di-(4-methoxypropylamino-1,2,2,6,6-pentamethylpiperidyl)-1,3,5-triazin und 1,2-Bis-(3-aminopropylamino)ethan, Kondensationsprodukt aus 2-Chlor-4,6-di-(4-n-butylamino-1,2,2,6,6-pentamethylpiperidyl)-1,3,5-triazin und 1,2-Bis-(3-aminopropylamino)ethan, Umsetzungsprodukte von 2-Chlor-4,6-di-(4-n-butylamino-2,2,6,6-tetramethylpiperidyl)-1,3,5-triazin mit ein- oder mehrwertigen Aminen, wobei zwischen einem und allen aktiven H-Atomen am Amin ersetzt werden, wie mit Ethylendiamin, Diethylentriamin, Triethylentetramin, Hexamethylendiamin, 1,2-Bis-(3-aminopropylamino)äthan, Umsetzungsprodukte von 2-Chlor-4,6-di-(4-n-butylamino-1,2,2,6,6-penta-methylpiperidyl)-1,3,5-triazin mit ein- oder mehrwertigen Aminen, wobei zwischen einem und allen aktiven H-Atomen am Amin ersetzt werden, wie mit Ethylendiamin, Di-ethylentriamin, Triethylentetramin, Hexamethylendiamin, 1,2-Bis-(3-aminopropyl-amino)ethan, Umsetzungsprodukte von 2-Chlor-4,6-di-(4-n-butylamino-2,2,6,6-tetra-methyl-piperidyl)-1,3,5-triazin und 4-tert-Octylamino-2,6-dichlor-1,3,5-s-triazin mit ein- oder mehrwertigen Aminen, wobei zwischen einem und allen aktiven H-Atomen am Amin ersetzt werden, wie mit Ethylendiamin, Diethylentriamin, Triethylentetramin, Hexamethylendiamin, 1,2-Bis-(3-aminopropylamino)ethan, Umsetzungsprodukte von 2-Chlor-4,6-di-(4-n-butylamino-1,2,2,6,6-pentamethylpiperidyl)-1,3,5-triazin und 4-tert-Octylamino-2,6-dichlor-1,3,5-s-triazin mit ein- oder mehrwertigen Aminen, wobei zwischen einem und allen aktiven H-Atomen am Amin ersetzt werden, wie mit Ethylen-diamin, Diethylentriamin, Triethylentetramin, Hexamethylendiamin, 1,2-Bis-(3-amino-propylamino)ethan, Umsetzungsprodukte von 2-Chlor-4,6-di-(4-n-butylamino-2,2,6,6-tetramethylpiperidyl)-1,3,5-triazin und 4-(4-n-Butylamino-2,2,6,6-tetramethyl-piperidyl)-2,6-dichlor-1,3,5-s-triazin mit ein- oder mehrwertigen Aminen, wobei zwischen einem und allen aktiven H-Atomen am Amin ersetzt werden, wie mit Ethylendiamin, Diethylen-triamin, Triethylentetramin, Hexamethylendiamin, 1,2-Bis-(3-aminopropylamino)-ethan, Umsetzungsprodukte von 2-Chlor-4,6-di-(4-n-butylamino-1,2,2,6,6-pentamethyl-piperidyl)-1,3,5-triazin und 4-(4-n-Butylamino-2,2,6,6-tetramethylpiperidyl)-2,6-dichlor-1,3,5-s-triazin mit ein- oder mehrwertigen Aminen, wobei zwischen einem und allen aktiven H-Atomen am Amin ersetzt werden, wie mit Ethylendiamin, Diethylentriamin, Triethylentetramin, Hexamethylendiamin, 1,2-Bis-(3-aminopropylamino)ethan, Konden-sationprodukt aus 1,2-Bis(3-aminopropylamino)-ethan und 2,4,6-Trichlor-1,3,5-triazin sowie 4-Butylamino-2,2,6,6-tetramethylpiperidin, N-(2,2,6,6-tetramethyl-4-piperidyl)-n-dodecylsuccinimid, N-(1,2,2,6,6-pentamethyl-4-piperidyl)-n-dodecylsuccinimid, 8-Acetyl-3-dodecyl-7,7,9,9-tetramethyl-1,3,8-triazaspiro[4,5]decan-2,4-dion, Oligomerisiertes 2,2,4,4-Tetramethyl-20-(oxiranylmethyl)-7-oxa-3,20-diaza-dispiro[5.1.11.2]heneicosan-21-on, Oligomerisiertes 1,2,2,4,4-Pentamethyl-20-(oxiranylmethyl)-7-oxa-3,20-diazadispiro-[5.1.11.2]heneicosan-21-on, Oligomerisiertes 1-Acetyl-2,2,4,4-tetramethyl-20-(oxiranylmethyl)-7-oxa-3,20-diaza-dispiro[5.1.11.2]heneicosan-21-on, 3-Dodecyl-1-(2,2,6,6-tetramethyl-4-piperidyl)pyrrolidin-2,5-dion, 3-Dodecyl-1-(1,2,2,6,6-pentamethyl-4-piperidyl)-pyrrolidin-2,5-dion, 2,2,4,4-Tetramethyl-7-oxa-3,20-diazadispiro-[5.1.11.2]-heneicosan-21-on, 2,2,4,4-Tetramethyl-7-oxa-21-oxo-3,20-diazadispiro-[5.1.11.2]-hen-eicosan-3-propansäure-dodecylester, 2,2,4,4-Tetramethyl-7-oxa-21-oxo-3,20-diaza-dispiro-[5.1.11.2]-heneicosan-3-propansäuretetradecylester, 2,2,3,4,4-Pentamethyl-7-oxa-3,20-diazadispiro-[5.1.11.2]heneicosan-21-on, 2,2,3,4,4-Penta-methyl-7-oxa-21-oxo-3,20-diaza-dispiro-[5.1.11.2]heneicosan-3-propansäuredodecylester, 2,2,3,4,4-Pentamethyl-7-oxa-21-oxo-3,20-diazadispiro-[5.1.11.2]-heneicosan-3-propansäure-tetradecylester, 3-Acetyl-2,2,4,4-tetramethyl-7-oxa-3,20-diazadispiro-[5.1.11.2]heneico-san-21-on, 3-Acetyl-2,2,4,4-tetramethyl-7-oxa-21-oxo-3,20-diaza-dispiro-[5.1.11.2]hen-eicosan-3-propansäure-dodecylester, 3-Acetyl-2,2,4,4-tetramethyl-7-oxa-21-oxo-3,20-diazadispiro-[5.1.11.2]heneicosan-3-propansäuretetradecylester, 1,1',3,3',5,5'-Hexa-hydro-2,2',4,4',6,6'-hexaaza-2,2',6,6'-bismethano-7,8-dioxo-4,4'-bis-(1,2,2,6,6-penta-methyl-4-piperidyl)-biphenyl, Poly-N,N'-bis-(2,2,6,6-tetramethyl-4-piperidyl)-1,8-diaza-decyclen, Additionsverbindung von 2,2,6,6-Tetramethyl-4-allyloxy-piperidin und Poly-methylhydrogensiloxan (Molmasse bis 4000), Additionsverbindung von 1,2,2,6,6-Pen-tamethyl-4-allyloxypiperidin und Polymethylhydrogensiloxan (Molmasse bis 4000), N,N'-Diformyl-N,N'-bis-(2,2,6,6-tetramethyl-4-piperidinyl)-hexamethylendiamin, N,N'-Di-formyl-N,N'-bis-(1,2,2,6,6-pentamethyl-4-piperidinyl)-hexamethylendiamin, 5,11-Bis-(2,2,6,6-tetramethyl-4-piperidinyl)-3,5,7,9,11,13-hexaaza-tetracyclo-[7.4.0.0^{2,7}.1^{3,13}]-tetradecan-8,14-dion, 5,11-Bis-(1,2,2,6,6-pentamethyl-4-piperidinyl)-3,5,7,9,11,13-hexaazatetracyclo-[7.4.0.0^{2,7}.1^{3,13}]-tetradecan-8,14-dion, [(4-Methoxyphenyl)-methylen]-propandisäure-bis-(2,2,6,6-tetramethyl-4-piperidinyl)-ester, [(4-Methoxyphenyl)-methyl-en]-propandisäure-bis-(1,2,2,6,6-pentamethyl-4-piperidinyl)-ester, 2,4,6-Tris-(N-cyclohexyl-N-[2-(3,3,4,5,5-pentamethyl-piperazinon-1-yl)-ethyl]-amino)-1,3,5-triazin, Copolymerisat aus Styrol mit Methylstyrol und Maleinsäureanhydrid umgesetzt mit 4-Amino-2,2,6,6-tetramethylpiperidin und Octadecylamin, Copolymerisat aus Styrol mit α-Methylstyrol und Maleinsäureanhydrid umgesetzt mit 4-Amino-1,2,2,6,6-pentamethylpiperidin und Octadecylamin, Polycarbonat mit 2,2'-[(2,2,6,6-Tetramethyl-4-piperidinyl)-imino]-bis-[ethanol] als Diolkomponente, Polycarbonat aus 2,2'-(1,2,2,6,6-Pentamethyl-4-piperidinyl)-imino]-bis-[ethanol] als Diolkomponente, Copolymer aus Maleinsäureanhydrid und einem α-Olefin bis C30 umgesetzt mit 4-Amino-2,2,6,6-tetramethylpiperidin, Copolymer aus Maleinsäureanhydrid und einem α-Olefin bis C₃₀ umgesetzt mit 1-Acetyl-4-amino-2,2,6,6-tetramethylpiperidin, Copolymer aus Maleinsäureanhydrid und einem α-Olefin bis C₃₀ umgesetzt mit 4-Amino-1,2,2,6,6-pentamethylpiperidin, sowie die N-Alkyl- und N-Aryl-oxyderivate der oben genannten Verbindungen mit freien NH-Gruppen am Piperidin, speziell α-Methylbenzyloxy- und Alkyloxy- von C₁ bis C₁₈.
2.7 Oxalsäurediamide, wie z.B. 4,4'-Di-octyloxyoxanilid, 2,2'-Diethoxyoxanilid, 2,2'-Dioctyloxy-5,5'-di-tert-butyl-oxanilid, 2,2'-Di-dodecyloxy-5,5'-di-tert-butyl-oxanilid, 2-Ethoxy-2'-ethyloxanilid, N,N'-Bis-(3-dimethylaminopropyl)-oxalamid, 2-Ethoxy-5-tert-butyl-2'-ethyloxanilid und dessen Gemisch mit 2-Ethoxy-2'-ethyl-5,4'-di-tert-butyl-oxanilid, Gemische von o- und p-Methoxy- sowie von o- und p-Ethoxy-di-substituierten Oxaniliden.
2.8. 2-(2-Hydroxyphenyl)-1,3,5-triazine, wie z.B. 2,4,6-Tris-(2-hydroxy-4-octyloxyphenyl)-1,3,5-triazin, 2-(2-Hydroxy-4-octyloxyphenyl)-4',6-bis-(2',4-dimethylphenyl)-1,3,5-triazin, 2-(2,4-Dihydroxyphenyl)-4,6-bis-(2,4-dimethylphenyl)-1,3,5-triazin, 2-(2,4-Dihydroxy-phenyl)-4,6-bis-(2,4-dimethylphenyl)-1,3,5-triazin, 2,4-Bis-(2-hydroxy-4-propyloxy-phenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethyl-phenyl)-1,3,5-triazin, 2-[2-Hydroxy-4-(2-hydroxy-3-butyloxypropyloxy)phenyl]-4,6-bis-(2,4-dimethylphenyl)-1,3,5-triazin, 2-[2-hydroxy-4-(2-hydroxy-3-octyloxypropyloxy)-phenyl]-4,6-bis(2,4-dimethylphenyl)-1,3,5-triazin, 2-(2-Hydroxy-4-tridecyloxyphenyl)-4,6-bis-(2,4-dimethylphenyl)-1,3,5-triazin, 2-[4-dodecyloxy/tridecyloxy-2-hydroxyprop-oxy)-2-hydroxy-phenyl]-4,6-bis(2,4-dimethylphenyl)-1,3,5-triazin, 2-[2-Hydroxy-4-(2-hy-droxy-3-dodecyloxypropoxy)phenyl]-4,6-bis(2,4-dimethylphenyl)-1,3,5-triazin, 2-(2-Hy-droxy-4-hexyloxy)phenyl-4,6-diphenyl-1,3,5-triazin, 2-(2-Hydroxy-4-methoxyphenyl)-4,6-diphenyl-1,3,5-triazin, 2,4,6-Tris[2-hydroxy-4-(3-butoxy-2-hydroxypropoxy)-phenyl]-1,3,5-triazin, 2-(2-Hydroxyphenyl)-4-(4-methoxyphenyl)-6-phenyl-1,3,5-triazin.
3. Metalldesaktivatoren, wie z.B. N,N'-Diphenyloxalsäurediamid, N-Salicylal-N'-salicyloylhydrazin, N,N'-Bis-(salicyloyl)-hydrazin, N,N'-Bis-(3,5-di-tert-butyl-4-hydroxyphenylpropionyl)-hydrazin, 3-Salicyloylamino-1,2,4-triazol, Bis-(benzyliden)-oxalsäuredihydrazid, Oxanilid, Isophthalsäuredihydrazid, Sebacinsäurebisphenyl-hydrazid, N,N'-Diacetyladipinsäuredihydrazid, N,N'-Bis-salicyloyloxalsäuredihydrazid, N,N'-Bis-salicyloylthiopropionsäuredihydrazid.
4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearylpentaerythritdiphosphit, Tris-(2,4-di-tert-butylphenyl)-phosphit, Diisodecylpentaerythritdiphosphit, Bis-(2,4-di-tert-butylphenyl)-pentaerythritdiphosphit, Bis-(2,6,-di-tert-butyl-4-methylphenyl)-pentaerythritdiphosphit, Bis-isodecyloxy-pentaerythritdiphosphit, Bis-(2,4-di-tert-butyl-6-methylphenyl)-pentaerythritdiphosphit, Bis-(2,4,6-tri-tert-butylphenyl)-pentaerytritdiphosphit, Tristearyl-sorbit-triphosphit, Tetrakis-(2,4-di-tert-butylphenyl)-4,4'-biphenylendiphosphonit, 6-lsooctyloxy-2,4,8,10-tetra-tert-butyl-12H-di-benz[d,g]-1,3,2-dioxaphosphocin, 6-Fluor-2,4,8,10-tetra-tert-butyl-12-methyl-dibenz[d,g]-1,3,2-dioxaphophocin, Bis-(2,4-di-tert-butyl-6-methylphenyl)-methylphosphit, Bis-(2,4-di-tert-butyl-6-methylphenyl)-ethylphosphit, Tris(2-tert-butyl-4-thio(2'-methyl-4'-hydroxy-5'-tert-butyl)-phenyl-5-methyl)-phenylphosphit, 2,2',2"-Nitrilo[triethyl-tris(3,3',5,5'-tetra-tert-butyl-1,1'-biphenyl-2,2'-diyl)phosphit], Bis[2-methyl-4,6-bis(1,1-dimethylethyl)phenol]phosphorigsäureethylester.
5. Hydroxylamine, wie z. B. N,N-Dibenzylhydroxylamin, N,N-Diethylhydroxylamin, N,N-Dioctylhydroxylamin, N,N-Dilaurylhydroxylamin, N,N-Ditetradecylhydroxylamin, N,N-Dihexadecylhydroxylamin, N,N-Dioctadecylhydroxylamin, N-Hexadecyl-N-octadecylhydroxylamin, N-Heptadecyl-N-octadecylhydroxylamin, N,N-Dialkylhydroxylaminen hergestellt aus hydriertem Talgfettamin.
6. Nitrone, wie z. B. N-Benzyl-alpha-phenylnitron, N-Ethyl-alpha-methylnitron, N-Octylalphaheptyl-nitron, N-Lauryl-alphaundecylnitron, N-Tetradecyl-alpha-tridecylnitron, N-Hexadecyl-alpha-pentadecylnitron, N-Octadecyl-alpha-heptadecyl-nitron, N-Hexadecylalpha-heptadecylnitron, N-Octadecyl-alpha-pentadecylnitron, N-Heptadecyl-alphaheptadecylnitron, N-Octa-decyl-alpha-hexadecylnitron, Nitrone abgeleitet von N,N-Dialkylhydroxylaminen, hergestellt aus hydrierten Talgfettaminen.
7. Zeolithe und Hydrotalcite, wie z. B. DHT 4A. Solche Hydrotalcite können nach der allgemeinen Formel

   [(M²⁺)₁₋ₓ(M³⁺)ₓ(OH)₂ (Aⁿ⁻)_{x/n}yH₂O],

   beschrieben werden, wobei
   - (M²⁺): Mg, Ca, Sr, Ba, Zn, Pb, Sn, Ni
   - (M³⁺): Al, B, Bi
   - An: ein Anion der Wertigkeit n
   - n: eine ganze Zahl von 1-4
   - x: einen Wert zwischen 0 und 0,5
   - y: einen Wert zwischen 0 und 2; bedeutet.
   - A: bedeutet OH⁻, Cl⁻, Br⁻, l⁻, ClO₄-, CH₃COO⁻, C₆H₅COO⁻, CO₃²⁻, SO₄²⁻, (OOC-COO)², (CHOHCOO)₂²⁻, HO(CHOH)₄CH₂COO⁻, C₂H₄(COO)₂²⁻, (CH₂COO)₂²⁻, CH₃CHOHCOO⁻, SiO₃²⁻, SiO₄⁴⁻, Fe(CN)₆³⁻, Fe(CN)₆⁴⁻, BO₃³⁻, PO₃³⁻, HPO₄²⁻,
   Bevorzugt eingesetzt werden Hydrotalcite, in welchen (M²⁺) für (Ca²⁺), (Mg²⁺) oder eine Mischung aus (Mg²⁺) und (Zn²⁺); (Aⁿ⁻) für CO₃²⁻, BO₃³⁻, PO₃³⁻ stehen. x hat einen Wert von 0 bis 0,5 und y hat einen Wert von 0 bis 2. Weiterhin können auch Hydrotalcite, die mit der Formel

   [(M²⁺)ₓ(Al³⁺)₂(OH)_{2x+6nz}(Aⁿ⁻)₂yH₂O]

   beschrieben werden können, eingesetzt werden. Hier steht (M²⁺) für Mg²⁺, Zn²⁺, bevorzugterweise jedoch Mg²⁺. (Aⁿ⁻) steht für ein Anion, besonders aus der Gruppe CO₃²⁻, (OOC-COO)²⁻, OH⁻ und S²⁻, wobei n die Wertigkeit des lons beschreibt.
   - y: steht für eine positive Zahl, bevorzugterweise zwischen 0 und 5, ganz besonders zwischen 0,5 und 5. x und z haben positive Werte, die bei x bevorzugt zwischen 2 und 6 liegen und bei z kleiner als 2 sein sollten. Besonders bevorzugt sind die Hydrotalcite der folgenden Formeln anzusehen:
   ${\text{Al}}_{\text{2}} {\text{O}}_{\text{3}} {\text{x 6MgO x CO}}_{\text{2}} {\text{x 12H}}_{\text{2}} \text{O,}$${\text{Mg}}_{\text{4.5}} {\text{Al}}_{\text{2}} {\text{(OH)}}_{\text{13}} {\text{x CO}}_{\text{3}} {\text{x 3.5H}}_{\text{2}} \text{O,}$${\text{4MgO x Al}}_{\text{2}} {\text{O}}_{\text{3}} {\text{x CO}}_{\text{2}} {\text{x 9H}}_{\text{2}} \text{O,}$${\text{4MgO x Al}}_{\text{2}} {\text{O}}_{\text{3}} {\text{x CO}}_{\text{2}} {\text{x 6H}}_{\text{2}} \text{O,}$${\text{ZnO x 3MgO x Al}}_{\text{2}} {\text{O}}_{\text{3}} {\text{x CO}}_{\text{2}} {\text{x 8-9H}}_{\text{2}} \text{O,}$${\text{ZnO x 3MgO x Al}}_{\text{2}} {\text{O}}_{\text{3}} {\text{x CO}}_{\text{2}} {\text{x 5-6H}}_{\text{2}} \text{O,}$${\text{Mg}}_{\text{4.5}} {\text{Al}}_{\text{2}} {\text{(OH)}}_{\text{13}} {\text{x CO}}_{\text{3}} \text{.}$ Hydrotalcite werden im Polymer bevorzugt in einer Konzentration von 0.01 bis 5 Gew.%, besonders von 0,2 bis 3 Gew.%, bezogen auf die gesamte Polymerzubereitung, eingesetzt.
8. Thiosynergisten, wie z. B. Thiodipropionsäuredilaurylester oder Thiodipropionsäuredistearylester.
9. Peroxidzerstörende Verbindungen wie z.B. Ester der ss-Thio-dipropionsäure, beispiels-weise der Lauryl-, Stearyl-, Myristyl- oder Tridecylester, Mercaptobenzimidazol, das Zinksalz des 2-Mercaptobenzimidazols, Zink-alkyldithiocarbamate, Zinkdibutyl-dithio-carbamat, Dioctadecylmonosulfid, Dioctadecyldisulfid, Pentaerythrit-tetrakis-(ss-do-decylmercapto)-propionat.
10. Polyamidstabilisatoren, wie z.B. Kupfersalze in Kombination mit Jodiden und/oder Phosphorverbindungen und Salze des zweiwertigen Mangans.
11. Basische Co-Stabilisatoren wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Behenat, Mg-Stearat, Na-Ricinoleat, K-Palmitat, Antimonbrenzcatechinat oder Zinnbrenzcatechinat, Alkali- und Erdalkalisalze sowie das Zinksalz oder das Aluminiumsalz von Milchsäure.
12. Nukleierungsmittel, wie z.B. anorganische Stoffe, wie z. B. Talk, Metalloxide, wie z. B. Titandioxid oder Magnesiumoxid, Phosphate, Carbonate oder Sulfate von vorzugsweise Erdalkalimetallen, organische Verbindungen, wie Mono- oder Polycarbonsäuren sowie auch ihrer Salze, wie z. B. 4-tert-Butylbenzoesäure, Adipinsäure; Diphenylessigsäure; Natriumsuccinat oder Natriumbenzoat; Acetale von aromatischen Aldehyden und polyfunktionellen Alkoholen wie z. B. Sorbitol, wie z.B. 1,3-2,4-Di(benziliden)-D-sorbitol, 1,3-2,4-Di(4-tolyliden)-D-sorbitol, 1,3-2,4-Di(4-ethylbenziliden)-D-sorbitol, polymere Verbindungen, wie z. B. ionische Copolymerisate ("Ionomere").
13. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Russ, Graphit, Holzmehl und andere Mehle oder Fasern anderer Naturprodukte, synthetische Fasern.
14. Sonstige Zusätze, wie z.B. Weichmacher, Gleitmittel, Emulgatoren, Pigmente, Rheologieadditive, Katalysatoren, Verlaufshilfsmittel, Optische Aufheller, Flammschutzmittel, Antistatika, Treibmittel.

Die Additive der allgemeinen Formel (1) oder die beschriebenen Kombinationen werden nach den allgemein üblichen Methoden in das organische Material, vorzugsweise in das Polymere eingearbeitet. Die Einarbeitung kann beispielsweise durch Einmischen oder Aufbringen der Verbindungen und gegebenenfalls weiterer Additive in oder auf das Polymere unmittelbar vor, während oder nach der Polymerisation oder in die Polymerschmelze vor oder während der Formgebung erfolgen. Auch durch Aufbringen der gelösten oder dispergierten Verbindungen auf das Polymere direkt oder Einmischen in eine Lösung, Suspension oder Emulsion des Polymeren, gegebenenfalls unter nachträglicher Verdunstung des Lösemittels, kann die Einarbeitung erfolgen. Die Verbindungen sind auch wirksam, wenn sie in ein bereits granuliertes Polymer nachträglich in einem gesonderten Verarbeitungsschritt eingebracht werden. Die Verbindungen der Formel (1) können auch in Form eines Masterbatches, der diese Verbindungen beispielsweise in einer Konzentration von 1 bis 75, vorzugsweise 2,5 bis 30 Gew.-% enthält, den zu stabilisierenden Polymeren zugesetzt werden.

### EXPERIMENTELLER TEIL

Die nachfolgenden Beispiele sollen die Erfindung näher erläutern, ohne diese in irgendeiner Weise näher einzuschränken.

### BEISPIELE 1 - 6

### Herstellung der neuen, erfindungsgemässen Verbindungen

In einem geschlossenen Reaktionsgefäss werden 0,35 mol 2,2,5,5-Tetramethylpiperidin-4-on unter Eiskühlung in 80 g Eisessig eingerührt. Das Gemisch wird in der verschlossenen Apparatur auf 60°C erwärmt. Anschliessend werden 0,40 mol Blausäure zugegeben. Die Reaktionsmischung wird 3 h bei 50°C gerührt. Nach Abkühlen auf etwa 20°C werden 0,70 mol Keton (bzw. Aldehyd) und 40 g Eisessig zugegeben. Unter Eiskühlung wird Chlorwasserstoff in die Lösung eingeleitet, bis das Reaktionsgemisch 0,80 mol Chlorwasserstoff aufgenommen hat. Die Mischung wird bei 80°C während 5 h gerührt und anschliessend im Vakuum von den flüchtigen Bestandteilen befreit. Nach Zugabe von 200 ml Aceton wird das Hydrochlorid der jeweiligen Zielverbindung abfiltriert, zwei mal mit je 50 ml Aceton nachgewaschen und im Vakuum bei 80°C getrocknet. Dieses Hydrochlorid wird mit 1000 ml Wasser verrührt und mittels 30%-iger Natronlauge auf pH 9 gestellt. Der entstehende Niederschlag wird filtriert, zwei mal mit je 50 ml Wasser nachgewaschen und im Vakuum bei 80°C getrocknet. Das gewünschte Produkt wird in Form eines weissen bis beigefarbenen feinkristallinen Pulvers isoliert.

**TABELLE 1**

| Zusammenstellung der Eigenschaften der neuen, erfindungsgemässen Verbindungen (Unter Bezugnahme auf Formel (1) gilt: X₁ = H; X₂ = H) | | | | |
|---|---|---|---|---|
| **Nr.** | **X**_{**3**} | **X**_{**4**} | **Schmelzpunkt** | **Ausbeute** |
| 1 | -CH₃ | -CH(CH₃)-(CH₂)-CH₃ | 140 - 144 °C | 77 % |
| 2 | -CH₃ | -(CH₂)-C(CH₃)₃ | 192 - 194 °C | 58 % |
| 3 | -CH₃ | -(CH₂)₂-CH(CH₃)₂ | 152 - 153 °C | 85 % |
| 4 | -(CH₂)-CH₃ | -(CH₂)-CH(CH₃)-(CH₂)-CH₃ | 114 - 116 °C | 68 % |
| 5 | H | -(CH₂)-CH(CH₃)₂ | 189 - 190 °C | 72 % |
| 6 | H | -CH(CH₃)₂ | 150 - 151 °C | 65 % |

### BEISPIELE 7 - 11

### Herstellung der nicht erfindungsgemässen Vergleichsverbindungen

Die Vergleichsverbindungen 7 - 11 werden nach der oben beschriebenen allgemeinen Methode hergestellt.

**TABELLE 2**

| Zusammenstellung der Eigenschaften der nicht erfindungsgemässen Vergleichsverbindungen (Unter Bezugnahme auf Formel (1) gilt: X₁ = H; X₂ = H) | | | | |
|---|---|---|---|---|
| **Nr.** | **X**_{**3**} | **X**_{**4**} | **Schmelzpunkt** | **Ausbeute** |
| 7 | Zusammen mit X₄ : | -(CH₂)₁₁- | 221 - 222 °C | 88 % |
| 8 | H | -(C₆H₅) | 264 - 265 °C | 83 % |
| 9 | H | -para-(C₆H₄)-C(CH₃)₃ | 254 - 264 °C | 80 % |
| 10 | H | -para-(C₆H₄)-O-(CH₃) | 223 - 225 °C | 75 % |
| 11 | -(CH₂)-CH₃ | -(C₆H₅) | 192 - 194 °C | 67 % |

### BEISPIEL 12

Schmelzstabilisierung von Polypropylen, das mit den betrachteten sterisch gehinderten Aminen zur Verlangsamung des thermischen Abbaus bei der Polymerverarbeitung stabilisiert ist. 100 Teile Polypropylen-Pulver, Typ Eltex P HL 001PF (Hersteller Solvay Polyolefines) wurden zusammen mit der Basisstabilisierung bestehend aus 0.05 Teilen Pentaerythrityl-tetrakis-3-(3,5-di-tert.butyl-4-hydroxyphenyl)propionat (Irganox 1010; Hersteller Ciba Specialty Chemicals) und 0.10 Teilen Ca-Stearat, 0.05 Teilen Tris-(2,4-di-tert.butylphenyl)-phosphit (Irgafos 168; Hersteller Ciba Specialty Chemicals) sowie 0.15 Teilen des sterisch gehinderten Piperidin-Derivats (z.B. Formel Typ 1) in einem Labormischer, Typ Major (Hersteller Kenwood) vermischt. Mit der so hergestellten Mischung wurde eine Vorextrusion in einem Einschnecken-Laborextruder (Schnecken-Durchmesser 25mm; UD=25; Kompression 1:3) (Hersteller Collin) mit 80 U/min. bei T=220°C durchgeführt. Die Mehrfachextrusion (1.-5. Passage) des Gemisches erfolgte im gleichen Extruder mit einer Drehgeschwindigkeit der Schnecke von 50 U/min. bei einer Temperatur aller Extruderzonen von T = 230 °C durch eine Düse vom Durchmesser 4 Millimetern. Der Schmelzindex wurde bei T = 230 °C mittels eines Schmelzindex-Messgeräts Typ 4105 des Herstellers Zwick unter Verwendung eines Normgewichts von 2.16kg bestimmt. Die Bestimmung der Farbe (Yl) des Granulats erfolgte mittels eines Spektrocolorimeters, Typ CM 3500d (Hersteller Minolta). Tabelle 3 gibt die Resultate der Mehrfachextrusion unter den geschilderten Bedingungen wieder.

**TABELLE 3**

| Mehrfachextrusion von Polypropylen (3.Generation) bei T= 230°C; das Polymer enthält folgende Komponenten als Basis-Additivierung: 0.05% Pentaerythrityl-tetrakis-3-(3,5-di-tert.butyl-4-hydroxyphenyl)propionat (Irganox 1010; Hersteller Ciba Specialty Chemicals); 0.05% Tris-(2,4-di-tert.butylphenyl)-phosphit (Irgafos 168; Hersteller Ciba Specialty Chemicals) sowie 0.10% Calciumstearat; die Konzentration des sterisch gehinderten Piperidin-Derivats (HAS) beträgt jeweils 0.15%. | | | | | | | | | |
|---|---|---|---|---|---|---|---|---|---|
| **Vers.-** | **HAS** | **Melt** | **Flow** | **Index** | **(MFI)** | | **Farbe** | **(YI)** | |
| **Nr.** | | **Vorextr.** | **1.Extr.** | **3.Extr.** | **5.Extr.** | **Vorextr** | **1.Extr.** | **3.Extr.** | **5.Extr** |
| 1 | ohne HAS | 2.62 | 3.04 | 3.74 | 3.84 | 3.28 | 11.23 | 13.54 | 15.13 |
| 2 | Tin. 770 | 2.74 | 2.92 | 3.27 | 3.94 | 4.77 | 11.81 | 14.84 | 17.29 |
| 3 | Host. N 20 | 2.74 | 2.89 | 3.28 | 3.64 | 2.52 | 8.41 | 11.05 | 13.14 |
| 4 | Verbindg.4 | 2.96 | 2.97 | 3.01 | 3.21 | 4.09 | 9.33 | 7.79 | 6.8 |

### BEISPIEL 13

Künstliche Bewitterung von Polypropylen, das mit den betrachteten sterisch gehinderten Aminen zur Verlangsamung des UV-Abbaus stabilisiert ist. Nach der 1. Extrusion des in Beispiel 12 beschriebenen Versuchs wurden Proben mittels einer Heizpresse, Typ Fontjne bei einer Temperatur T=230°C zunächst für 90 Sekunden bei einem Pressdruck von 50kN, anschliessend nach Entspannen für gleichfalls 90 Sekunden mit einem Pressdruck von 500 kN zu Filmen (Dicke ca.100 µm) verarbeitet. Die Belichtung erfolgte unter Zuhilfenahme eines Geräts UV-CON A (max. Wellenlänge λ=340nm) mit Belichtungszyklen von 24 Stunden bei einer Temperatur der Schwarztafeln von T=50 °C. Die Zersetzung der Probe wurde anhand der mittels IR-Spektroskopie (Gerät Typ FTS 155, Hersteller Bio-Rad) verfolgten Carbonylabsorption verfolgt. Hierbei wurde die Zeit (in Stunden) bestimmt, innerhalb der ein Anstieg der Carbonylabsorption um 0.3 Einheiten beobachtet wurde. Die Ergebnisse sind in Tab. 4 verzeichnet.

**TABELLE 4**

| Zeit der künstlichen Bewitterung, bis ΔCO > 0.3 Einheiten. | | | | | | |
|---|---|---|---|---|---|---|
| Verbindg. | 4 | 7 | 8 | 9 | 10 | 11 |
| Zeit | 1760 h | 1532 h | 1218 h | 1034 h | 615 h | 1465 h |

Es ist klar sichtbar, dass sich die erfindungsgemässe Verbindung 4 deutlich von den nicht erfindungsgemässen Vergleichsverbindungen abhebt.

## Patentansprüche

1. Stabilisatoren der allgemeinen Formel (1) 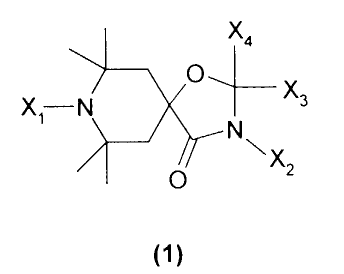 worin
X₁ Wasserstoff, eine C₁-C₂₂-Alkylgruppe; ein Sauerstoffradikal O*; -OH; -NO; -CH₂CN; Benzyl; Allyl; eine C₁-C₃₀-Alkoxygruppe; eine C₅-C₁₂-Cycloalkyloxygruppe; eine C₆-C₁₀-Aryloxygruppe, wobei der Arylrest zusätzlich noch durch eine C₁-C₅-Alkylgruppe oder ein Halogen oder eine Nitrogruppe substituiert sein kann; eine C₇-C₂₀-Arylalkyloxygruppe, wobei der Arylrest zusätzlich noch durch eine C₁-C₅-Alkylgruppe oder ein Halogen oder eine Nitrogruppe substituiert sein kann; eine C₃-C₁₀-Alkenylgruppe; eine C₃-C₆-Alkinylgruppe; eine C₁-C₁₀-Acylgruppe; Halogen; einen unsubstituierten oder durch C₁-C₄-Alkyl substituierten Phenylrest, bedeutet;
X₂ Wasserstoff, einen [CH₂-CH₂-C(O)-O-X₅]-Rest, einen [CH₂-C(CH₃)H-C(O)-O-X₅]-Rest; eine C₁-C₂₂-Alkylgruppe bedeutet;
X₃ und X₄ unabhängig voneinander Wasserstoff, einen unsubstituierten oder durch C₁-C₄-Alkyl substituierten Phenylrest oder eine C₁-C₂₂-Alkylgruppe, wobei mindestens einer der zwei Reste X₃ oder X₄ eine verzweigte C₄-C₂₂-Alkylgruppe, bedeuten; und worin
X₅ eine C₁-C₂₂-Alkylgruppe bedeutet.

2. Gemisch aus einer Verbindung der allgemeinen Formel (1) gemäss Anspruch 1 mit einer oder mehreren Stabilisatoren auf Basis sterisch gehinderter Amine der Formeln A1 bis A10, 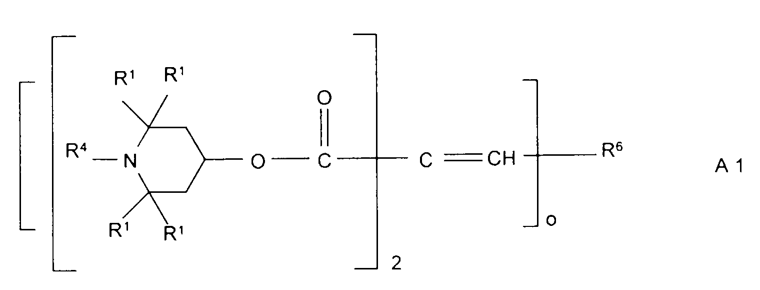 worin
R¹ Wasserstoff, C₅-C₇-Cycloalkyl oder eine C₁-C₁₂-Alkylgruppe und
R⁴ entweder Wasserstoff oder eine C₁-C₂₂-Alkylgruppe, ein Sauerstoffradikal O*, -OH, -NO-, CH₂CN, Benzyl, Allyl, eine C₁-C₃₀-Alkoxygruppe, eine C₅-C₁₂-Cycloalkyloxygruppe, eine C₆-C₁₀-Aryloxygruppe, wobei der Arylrest zusätzlich noch substituiert sein kann, eine C₇-C₂₀-Aralkyloxygruppe, wobei der Arylrest zusätzlich noch substituiert sein kann, eine C₃-C₁₀-Alkenylgruppe, eine C₃-C₆-Alkinylgruppe, eine C₁-C₁₀-Acylgruppe, Halogen, unsubstituiertes oder am Phenylring durch C₁-C₄-Alkyl substituiertes C₇-C₉-Phenylalkyl,
R⁶ einen ein oder mehrfach durch Wasserstoff, C₁-C₄-Alkyl, C₁-C₄-Alkoxy, Halogen, Cyan, Carboxy, Nitro, Amino, C₁-C₄ -Alkylamino, C₁-C₄-Dialkylamino, oder Acyl substituierten aromatischen Rest,
o 1 oder 2, bedeutet. 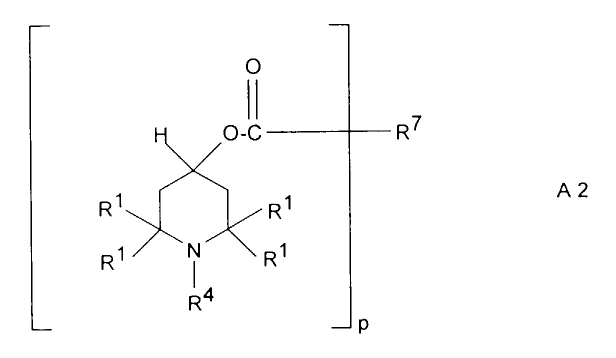
worin
R¹ und R⁴ die weiter oben angeführten Bedeutungen haben,
p 1 oder 2 und
für p = 1 R⁷ C₁-C₂₂-Alkyl, C₂-C₁₈-Oxaalkyl, C₂-C₁₈-Thiaalkyl, C₂-C₁₈-Azaalkyl oder C₂-C₈-Alkenyl bedeutet,
für p = 2 R⁷ C₁-C₂₂-Alkylen, C₂-C₁₈-Oxaalkylen, C₂-C₁₈-Thiaalkylen, C₂-C₁₈-Azaalkylen oder C₂-C₈-Alkenylen bedeutet. 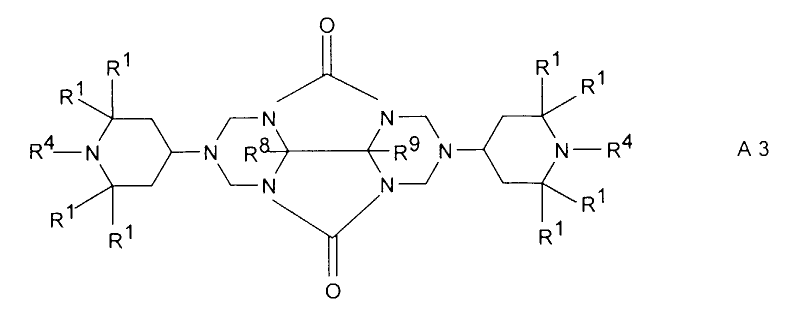
worin
R¹ und R⁴ die weiter oben angeführten Bedeutungen haben,
R⁸ und R⁹ unabhängig voneinander Wasserstoff, C₁-C₆-Alkyl, C₇-C₁₂-Aralkyl, -Aryl oder -Carbonsäureester,
R⁸ und R⁹ zusammen eine Tetra- oder Pentamethylgruppe bedeuten. 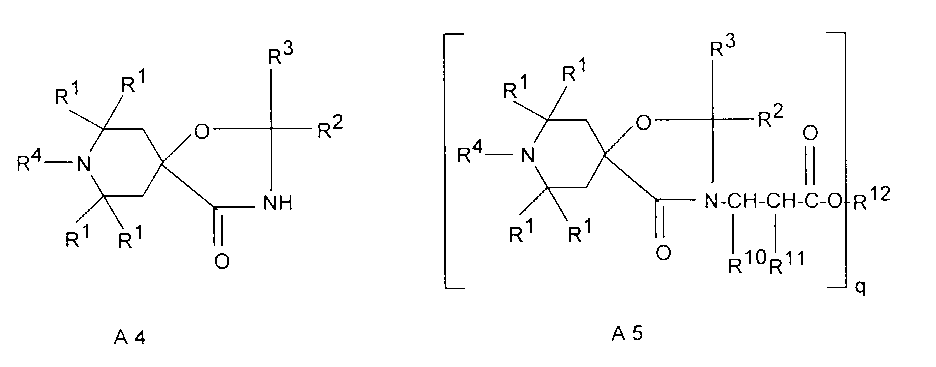
worin
R¹ und R⁴ die weiter oben angeführten Bedeutungen haben,
R² und R³ unabhängig voneinander ein Wasserstoffatom, eine C₁-C₁₈ -Alkylgruppe oder zusammen mit dem sie verbindenden C-Atom einen Ring der Ringgrösse 5 bis 13 oder zusammen mit dem sie bindenden C-Atom eine Gruppe der Formel (IV), 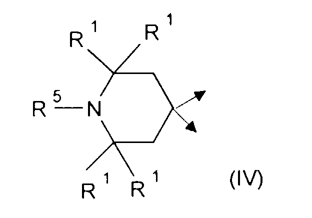
R⁴ und R⁵ unabhängig voneinander entweder Wasserstoff oder eine C₁-C₂₂-Alkylgruppe, ein Sauerstoffradikal O*, -OH, -NO, -CH₂CN, Benzyl, Allyl, eine C₁-C₃₀-Alkyloxygruppe, eine C₅-C₁₂-Cycloalkyloxygruppe, eine C₆-C₁₀-Aryloxygruppe, wobei der Arylrest zusätzlich noch substituiert sein kann, eine C₇-C₂₀-Arylalkyloxygruppe, wobei der Arylrest zusätzlich noch substituiert sein kann, eine C₃-C₁₀- Alkenylgruppe, eine C₃-C₆-Alkinylgruppe, eine C₁-C₁₀-Acylgruppe, Halogen, unsubstituiertes oder am Phenylring durch C₁-C₄-Alkyl substituiertes C₇-C₉-Phenylalkyl bedeuten,
q eine Zahl von 1 oder 2,
R¹⁰ Wasserstoff, Methyl, Phenyl oder Carb-C₁-C₂₁-Alkoxy,
R¹¹ Wasserstoff oder Methyl,
R¹² für q = 1 Wasserstoff, C₁-C₂₁-Alkyl, C₂-C₂₂-Alkenyl, C₅-C₁₂-Cycloalkyl, ein Radikal der Formel 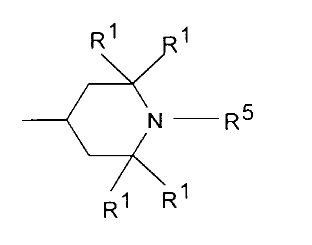
bedeutet, wobei R¹ und R⁵ die weiter oben angeführte Bedeutung haben, und
R¹² für q = 2 C₁-C₁₈-Alkylen, C₅-C₉-Cycloalkylen oder Arylen bedeutet. 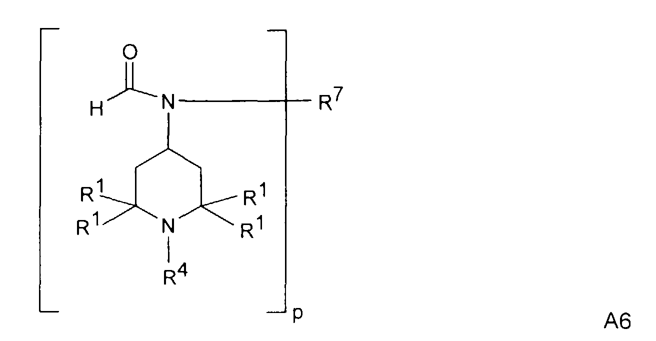
wobei R¹, R⁴, R⁷ und p die oben genannten Definitionen besitzen. 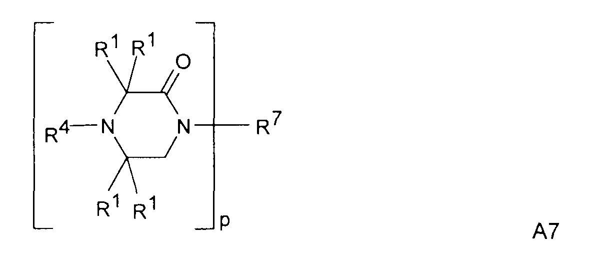 wobei R¹, R⁴, R⁷ und p die oben genannten Definitionen besitzen. 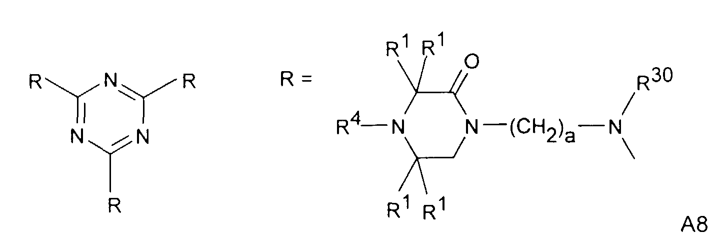 wobei
R¹, R⁴ die oben genannten Definitionen besitzen,
R³⁰ Wasserstoff, C₁-C₁₂-Alkyl, C₅-C₁₂-Cycloalkyl, Phenyl oder C₇-C₉-Phenylalkyl, und
a eine Zahl von 1 bis 10 bedeutet. 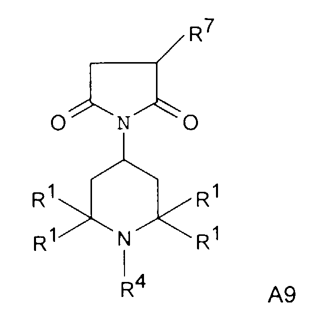
wobei
R¹ und R⁴ die obige Bedeutung hat und
R⁷ die in der Formel A2 für p=1 definierte Bedeutung hat.
Ein Produkt A10 erhältlich durch Umsetzung eines Polyamins der Formel A10a mit Formel A10b: 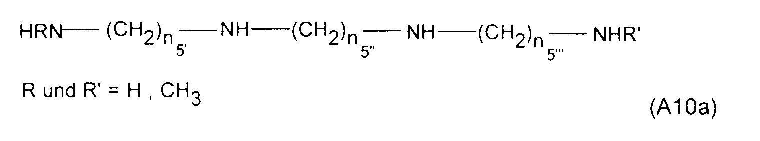 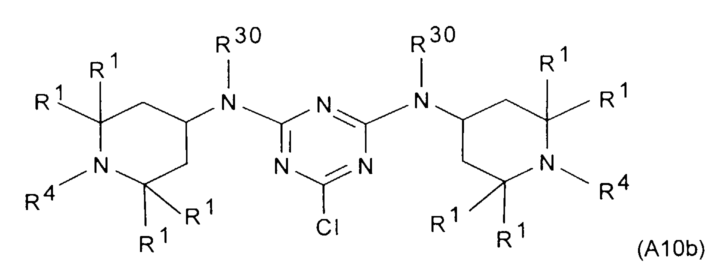 wobei
R¹, R⁴ und R³⁰ die oben angegebene Bedeutung haben,
n_{5'}, n_{5"} und n_{5"'} unabhängig voneinander eine Zahl von 2 bis 12 ist.

3. Gemisch aus einer Verbindung der allgemeinen Formel (1) gemäss Anspruch 1 mit einer oder mehreren polymeren HALS-Verbindungen der Formeln B1 bis B7 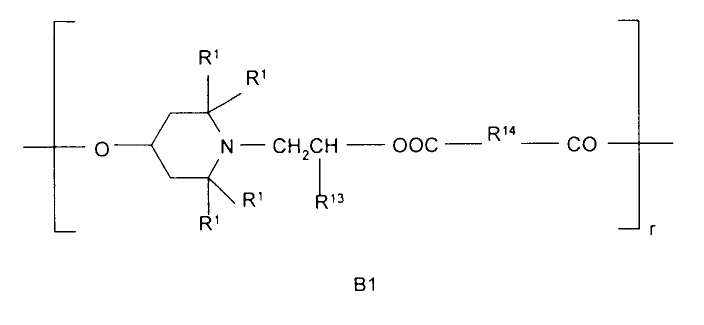 worin
R¹ Wasserstoff, C₅-C₇-Cycloalkyl oder eine C₁-C₁₂-Alkylgruppe,
R¹³ Wasserstoff oder Methyl,
R¹⁴ eine direkte Bindung oder C₁-C₁₀-Alkylen und
r eine Zahl von 2 bis 50 bedeutet. 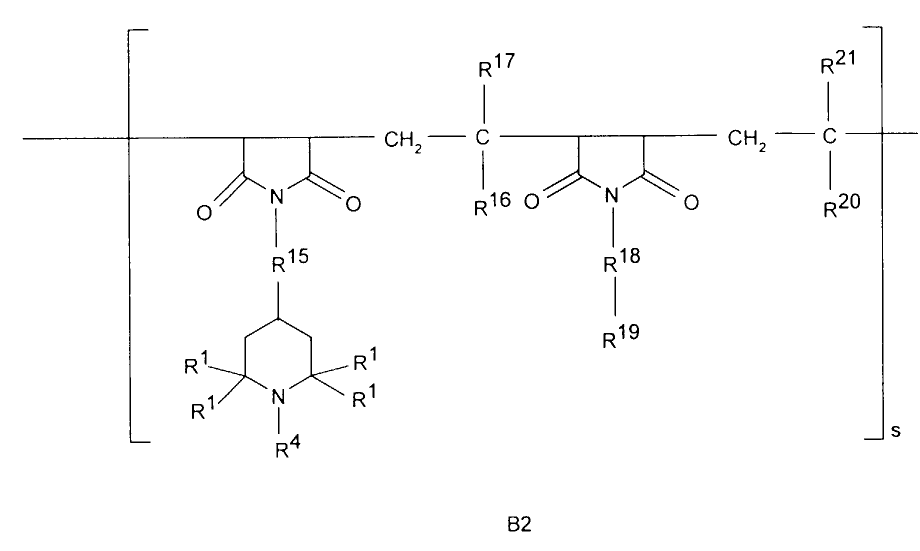
wobei
R¹ und R⁴ die weiter oben angegebenen Bedeutungen haben,
R¹⁵ und R¹⁸ unabhängig voneinander eine direkte Bindung oder eine Gruppe -N(R²²)-CO- R²³-CO-N(R²⁴)-,
R²² und R²⁴ unabhängig voneinander Wasserstoff, C₁-C₈-Alkyl, C₅-C₁₂-Cycloalkyl, Phenyl, C₇-C₉-Phenylalkyl, oder eine Gruppe der Formel 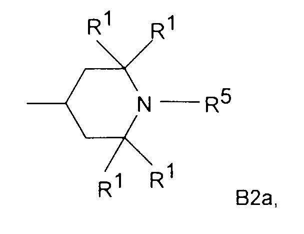
R²³ eine direkte Bindung oder C₁-C₄-Alkylen,
R¹⁶, R¹⁷, R²⁰, R²¹ unabhängig voneinander Wasserstoff, C₁-C₃₀-Alkyl, C₅-C₁₂-Cycloalkyl, Phenyl, oder eine Gruppe der Formel B2a,
R¹⁹ Wasserstoff, C₁-C₃₀-Alkyl, C₅-C₁₂-Cycloalkyl, C₇-C₉-Phenylalkyl, Phenyl oder eine Gruppe der Formel B2a bedeuten und
s eine Zahl von 1 bis 50 ist. 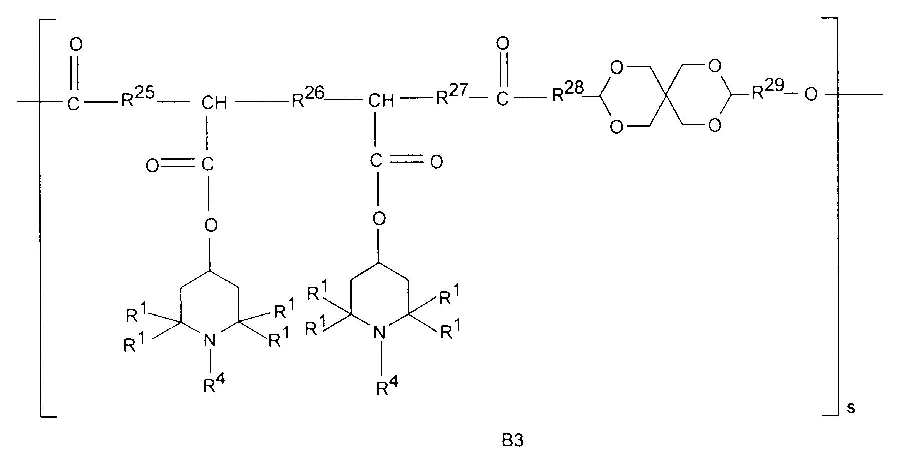
wobei
R¹, R⁴ und s die oben angegebenen Bedeutungen haben,
R²⁵, R²⁶, R²⁷, R²⁸ und R²⁹ unabhängig voneinander eine direkte Bindung oder C₁-C₁₀-Alkylen sind.
Ein Produkt B4 erhältlich durch Umsetzung eines durch Reaktion eines Polyamins der Formel B4a mit Cyanursäurechlorid erhaltenen Produktes mit einer Verbindung der Formel B4b, 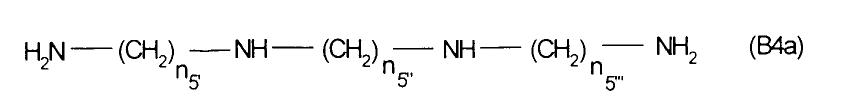 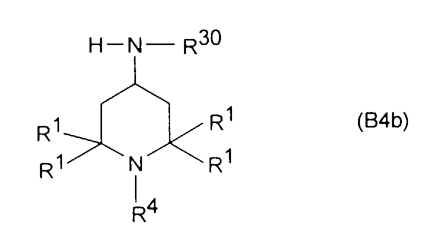 wobei
R¹ und R⁴ die weiter oben angegebenen Bedeutungen haben,
n_{5'}, n_{5"} und n_{5"'} unabhängig voneinander eine Zahl von 2 bis 12 sind,
R³⁰ die oben angegebene Bedeutung hat; wobei B4 eine Verbindung der Formel B4-1, B4-2, B4-3 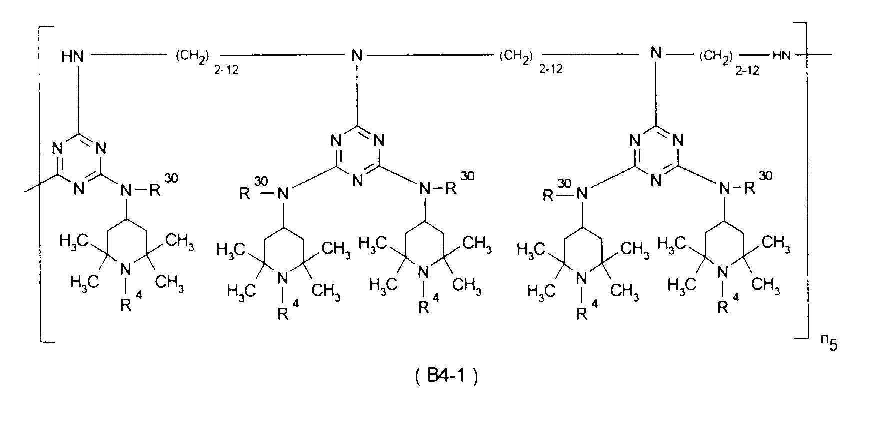 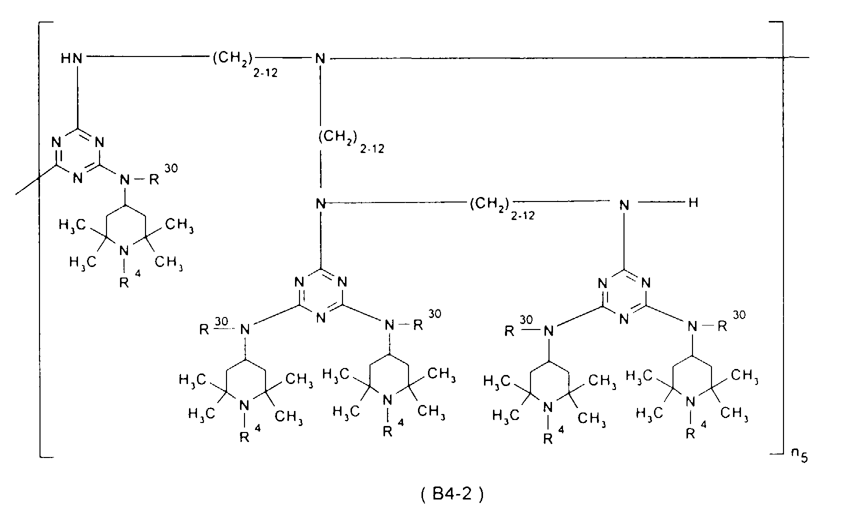 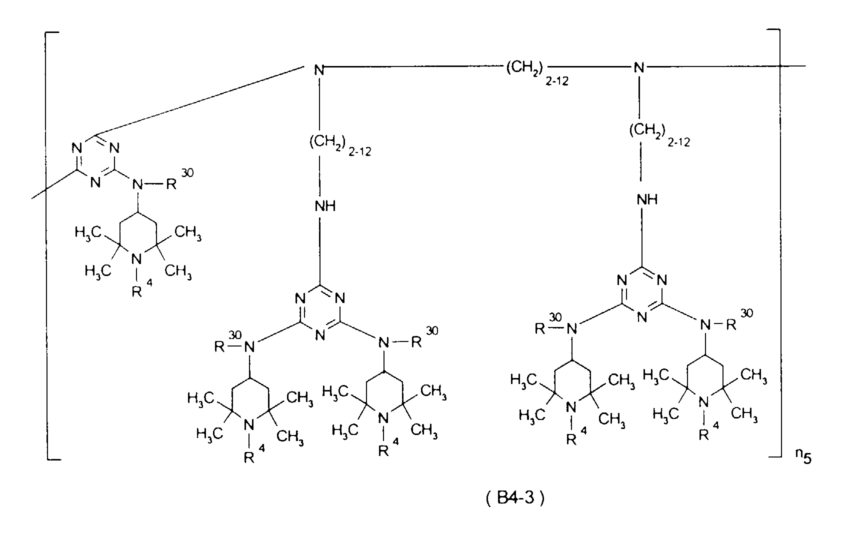
oder ein Gemisch daraus darstellt, worin
n₅ 1 bis 20,
R⁴ und R³⁰ die oben angegebene Bedeutung haben; 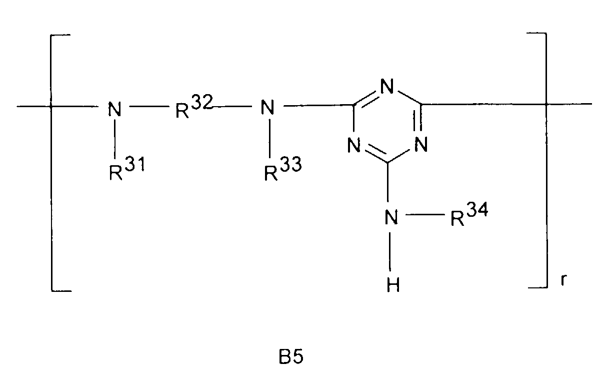
wobei
r die bei Formel B1 angegebene Bedeutung hat,
R³¹, R³³ und R³⁴ unabhängig voneinander Wasserstoff, C₁-C₁₂-Alkyl, C₅-C₁₂-Cycloalkyl, durch C₁-C₄-Alkyl substituiertes C₅-C₁₂-Cycloalkyl, Phenyl, durch -OH und/oder C₁-C₁₀-Alkyl substituiertes Phenyl, C₇-C₉-Phenylalkyl, am Phenylrest durch -OH und/oder C₁-C₁₀-Alkyl substituiertes C₇-C₉-Phenylkalkyl oder eine Gruppe der Formel B5a bedeuten, 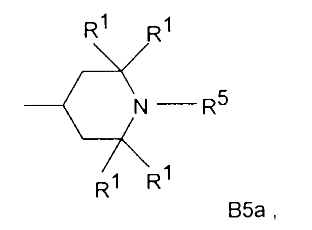 wobei R¹ und R⁵ die oben angegebenen Bedeutungen haben, und
R³² C₂-C₁₈-Alkylen, C₅-C₇-Cycloalkylen oder C₁-C₄-Alkylendi(C₅-C₇-Cyloalkylen) ist, oder
die Reste R³¹, R³² und R³³ zusammen mit den Stickstoffatomen, an die sie gebunden sind, einen 5- bis 10-gliedrigen heterocyclischen Ring bilden, und wobei mindestens einer der Reste R³¹, R³³ und R³⁴ eine Gruppe der Formel B5a darstellt. 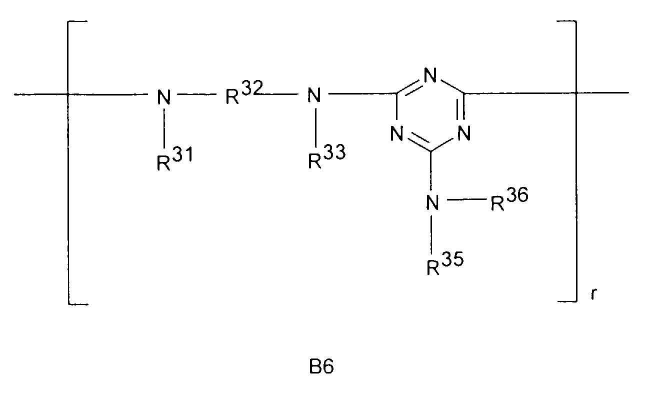
worin
R³¹, R³², R³³ und r die oben angegebenen Bedeutungen haben,
R³⁵ und R³⁶ unabhängig voneinander die Definition von R³⁴ haben kann, oder
R³⁵ und R³⁶ zusammen mit dem Stickstoffatom, an das sie gebunden sind, einen 5- bis 10-gliedrigen heterozyklischen Ring bilden, wobei dieser zusätzlich zum Stickstoff-Heteroatom noch ein oder mehrere Heteroatome, vorzugsweise ein Sauerstoffatom, enthalten kann und mindestens einer der Reste R³¹, R³³, R³⁵ und/oder R³⁶ eine Gruppe der Formel (B5a) darstellt. 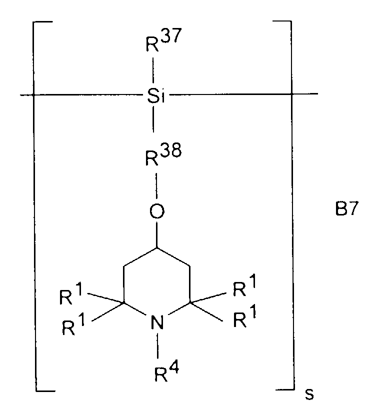
wobei
R¹ und R⁴ die weiter oben angegebene Bedeutung haben,
s die in Formel B3 angegebenen Bedeutung hat,
R³⁷ C₁-C₁₀-Alkyl, C₅-C₁₂-Cycloalkyl, durch C₁-C₄-Alkyl substituiertes C₅-C₁₂-Cycloalkyl, Phenyl oder durch C₁-C₁₀-Alkyl substituiertes Phenyl ist, und
R³⁸ C₃-C₁₀-Alkylen darstellt.

4. Mischung aus Verbindungen der allgemeinen Formel (1) gemäss Anspruch 1 und einem oder mehreren Phosphor-Stabilisatoren der allgemeinen Formeln C1 bis C7 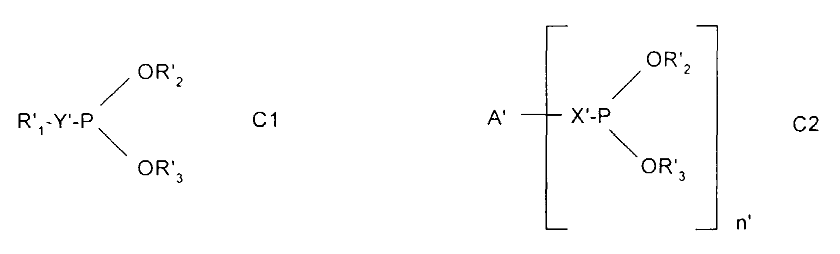 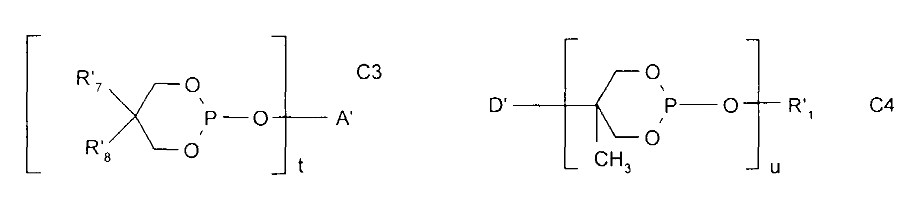 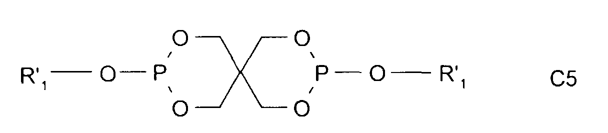 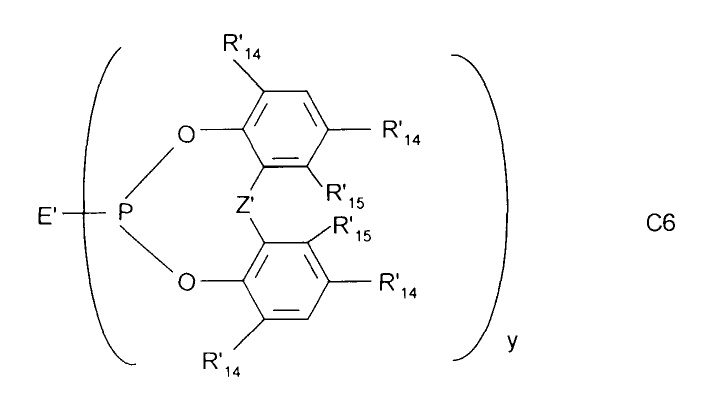 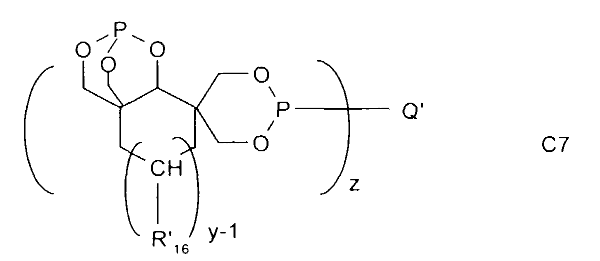 worin die Indices ganzzahlig sind und n' für 2, 3 oder 4; u für 1 oder 2; t für 2 oder 3; y für 1, 2 oder 3; und z für 1 bis 6 steht; A', wenn n' 2 ist, Alkylen mit 2 bis 18 Kohlenstoffatomen; durch -S-, -O- oder -NR'₄- unterbrochenes Alkylen mit 2 bis 12 Kohlenstoffatomen; ein Rest einer der Formeln 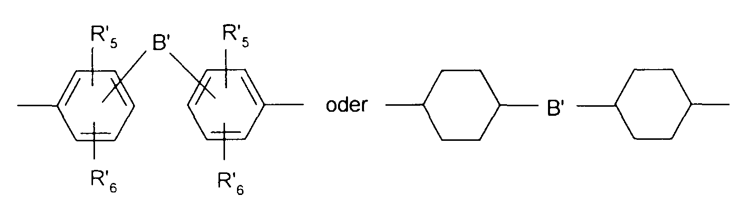 oder Phenylen ist;
A' wenn n' 3 ist, ein Rest der Formel -CᵣH₂ᵣ₋₁ ist;
A' wenn n' 4 ist, den Rest der Formel C(CH2)4- bedeutet;
A" die Bedeutung von A', wenn n' 2 ist, hat;
B' einen Rest der Formel -CH₂-; -CHR'₄-; -CR'₁R'₄-; -S- oder eine direkte Bindung darstellt; oder C₅-C₇-Cycloalkyliden; oder mit 1 bis 4 C₁-C₄-Alkylresten in Position 3, 4 und/oder 5 substituierters Cyclohexyliden bedeutet,
D' wenn u 1 ist, Methyl und, wenn u 2 ist, -CH₂OCH₂- bedeutet;
E' wenn y 1 ist, Alkyl mit 1 bis 18 Kohlenstoffatomen, Phenyl, ein Rest der Formel -OR'₁ oder Halogen ist;
E' wenn y 2 ist, ein Rest der Formel -O-A"-O- ist;
E' wenn y 3 ist, ein Rest der Formel R'₄C(CH₂O)₃- oder N(CH₂-CH₂-O-)₃ ist;
Q' für den Rest eines mindestens z-wertigen Alkohols oder Phenols steht, wobei dieser über das (die) alkoholische(n) bzw. phenolische(n) O-Atom(e) an das (die) P-Atom(e) gebunden ist;
R'₁, R'₂ und R'₃ unabhängig voneinander Alkyl mit 1 bis 30 Kohlenstoffatomen; mit Halogen, -COOR₄', -CN oder -CONR₄'R₄' substituiertes Alkyl mit 1 bis 18 Kohlenstoffatomen; durch -S-, -O- oder -NR'₄- unterbrochenes Alkyl mit 2 bis 18 Kohlenstoffatomen; Phenyl-C₁-C₄-alkyl; Cycloalkyl mit 5 bis 12 Kohlenstoffatomen; Phenyl oder Naphthyl; mit Halogen, 1 bis 3 Alkylresten oder Alkoxyresten mit insgesamt 1 bis 18 Kohlenstoffatomen oder mit Phenyl-C₁-C₄-Alkyl substituiertes Phenyl oder Naphthyl; oder ein Rest der Formel 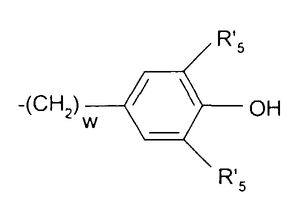 sind, worin w eine ganze Zahl aus dem Bereich 3 bis 6 bedeutet;
R'₄ beziehungsweise die Reste R'₄ unabhängig voneinander Wasserstoff, Alkyl mit 1 bis 18 Kohlenstoffatomen, Cycloalkyl mit 5 bis 12 Kohlenstoffatomen, oder Phenylalkyl mit 1 bis 4 Kohlenstoffatomen im Alkylteil;
R'₅ und R'₆ unabhängig voneinander Wasserstoff, Alkyl mit 1 bis 8Kohlenstoffatomen oder Cycloalkyl mit 5 oder 6 Kohlenstoffatomen sind;
R'₇ und R'₈ im Fall t = 2 unabhängig voneinander C₁-C₄-Alkyl oder zusammen einen 2,3-Dehydropentamethylenrest darstellen; und
R'₇ und R'₈ im Fall t = 3, Methyl bedeuten;
die Substituenten R'₁₄ unabhängig voneinander Wasserstoff, Alkyl mit 1 bis 9 Kohlenstoffatomen oder Cyclohexyl sind;
die Substituenten R'₁₅ unabhängig voneinander Wasserstoff oder Methyl; und
R'₁₆ Wasserstoff oder C₁-C₄-Alkyl darstellt und im Fall, dass mehrere Reste R'₁₆ vorhanden sind, die Reste R'₁₆ gleich oder verschieden sind;
X' und Y' jeweils eine direkte Bindung oder -O- darstellen; und
z eine direkte Bindung; -CH₂-; -C(R'₁₆)₂- oder -S- ist.

5. Mischung aus Verbindungen der allgemeinen Formel (1) gemäss Anspruch 1 und UV-Absorbern aus der Klasse der 2-Hydroxybenzophenone, 2-Hydroxyphenylbenztriazole, Zimtsäurederivate oder der Oxanilide.

6. Mischung gemäss den Ansprüchen 1 bis 5, dadurch gekennzeichnet, dass der Anteil an Verbindungen der Formel (1) zwischen 1 und 99 Gew.-% liegt.

7. Mischung aus Verbindungen der allgemeinen Formel (1) gemäss Anspruch 1 und Zeolithen oder Hydrotalciten.

8. Mischung aus Verbindungen der allgemeinen Formel (1) gemäss Anspruch 1 allein oder in Mischungen gemäss den Ansprüchen 1 bis 4 und einem oder mehreren N,N-dialkylsubstituierten Hydroxylaminen.

9. Mischung aus Verbindungen der allgemeinen Formel (1) gemäss Anspruch 1 und einem oder mehreren basischen oder sonstigen säurebindenden Costabilisatoren aus der Gruppe der Metallcarboxylate, -oxide, -hydroxide, -carbonate, und/oder Zeolithe, und/oder Hydrotalcite.

10. Verwendung von Stabilisatoren gemäss den Ansprüchen 1 oder von Mischungen gemäss den Ansprüchen 2 bis 9 zum Stabilisieren von organischem Material.
